(19) Europäisches Patentamt
European Patent Office
Office européen des brevets

(11) **EP 3 956 663 B1**

(12) **EUROPEAN PATENT SPECIFICATION**

(45) Date of publication and mention
of the grant of the patent:
**12.02.2025 Bulletin 2025/07**

(21) Application number: **20716858.4**

(22) Date of filing: **14.04.2020**

(51) International Patent Classification (IPC):
***G01N 33/50*** *(2006.01)*

(52) Cooperative Patent Classification (CPC):
**G01N 33/5005; G01N 33/5038; G01N 33/5091**

(86) International application number:
**PCT/EP2020/060486**

(87) International publication number:
**WO 2020/212362 (22.10.2020 Gazette 2020/43)**

(54) **A METHOD OF PROFILING THE ENERGETIC METABOLISM OF A POPULATION OF CELLS**

VERFAHREN ZUR PROFILIERUNG DES ENERGETISCHEN STOFFWECHSELS IN ZELLEN-POPULATIONEN

PROCÉDÉ DE PROFILAGE DU MÉTABOLISME ÉNERGÉTIQUE DANS DES POPULATIONS DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2019 EP 19305485**

(43) Date of publication of application:
**23.02.2022 Bulletin 2022/08**

(73) Proprietors:
• **Institut National de la Santé et de la Recherche Médicale (INSERM)**
**75013 Paris (FR)**
• **CENTRE NATIONAL DE LA RECHERCHE SCIENTIFIQUE (CNRS)**
**75016 Paris (FR)**
• **Université d'Aix Marseille**
**13284 Marseille Cedex 07 (FR)**

(72) Inventors:
• **ARGÜELLO, Rafael**
**13288 MARSEILLE CEDEX 09 (FR)**
• **PIERRE, Philippe**
**13288 MARSEILLE CEDEX 09 (FR)**
• **COMBES, Alexis**
**83110 SANARY-SUR-MER (FR)**

(74) Representative: **Inserm Transfert**
**PariSanté Campus**
**10 rue d'Oradour-sur-Glane**
**75015 Paris (FR)**

(56) References cited:
**WO-A1-2017/153992    US-A- 5 962 244**
**US-A1- 2016 281 049**

• **ARVIND RAMANATHAN ET AL: "Perturbational Profiling of a Cell-Line Model of Tumorigenesis by Using Metabolic Measurements",** PROCEEDINGS OF THE NATIONAL ACADEMY OF SCIENCES OF THE UNITED STATES OF AMERICA, vol. 102, no. 17, 26 April 2005 (2005-04-26), US, pages 5992 - 5997, XP055485801, ISSN: 0027-8424, DOI: 10.1073/ pnas.0502267102
• **JIN ZHANG ET AL: "Measuring energy metabolism in cultured cells, including human pluripotent stem cells and differentiated cells",** NATURE PROTOCOLS, vol. 7, no. 6, 10 May 2012 (2012-05-10), GB, pages 1068 - 1085, XP055485767, ISSN: 1754-2189, DOI: 10.1038/ nprot.2012.048

**Description**

**FIELD:**

The present disclosure relates to a method of profiling the energetic metabolism of a population of cells.

**BACKGROUND:**

The energetic metabolism (EM) profile describes the main sources of energy and biochemical pathways from which cells depend on to produce ATP (dependency) and also their potential to exploit other alternatives (capacity). EM profile also determines the competence of cells to survive in different anatomic locations and upon exposure to intrinsic and extrinsic signaling cues. This information is central to understand the physiological function and cellular state of different cell types. Among others, cancer stem cells, tumoral cells, immune cells, neurons, have an EM profile that impact on their capacity to proliferate, differentiate, and perform their physiological functions. Moreover, EM profile is also key in tumors, as it allows to determine the susceptibility of transformed cells to inhibitors of particular metabolic pathways (Wallace et al., 2010) (Connolly et al., 2014; Ganeshan and Chawla, 2014; MacIver et al., 2013). Recently, studies on immuno-metabolism indicate that immune cells have a tightly controlled and cell type specific metabolic profile. This metabolic profile is acquired by the expression of particular genes cell that regulate EM and that are part of their differentiation program. Immune or cancer cell specific EM profile reflect the state of activation or differentiation and determine the competence of these cells to migrate, survive, or respond to different immunological challenges.

At the present, a revolution in the treatment of cancer is occurring due to recent results of clinical trials after immuno-therapies. These therapies exploit the cytotoxic potential of the adaptive immune responses to control cancer development (Assmann and Finlay, 2016; Dougan and Dranoff, 2009). These therapies aim to restore or generate an activated status of immune cells generally target cytotoxic CD8+ T cells directly or indirectly, and include checkpoint inhibitors, chimeric antigen receptors (CAR) T cells, Bi-specific T-cell engagers (BiTEs) and adoptive cell transfer (Newick et al., 2017; Page et al., 2014). The enthusiasm in these treatments, relies on their demonstrated ability to cure cancer in some patients, even when used as third-line therapy. However, this complete remission responses are only observed few patients. Such heterogeneous responses, have created the need of markers to better characterize the tumors and tumor infiltrating cells, in order to identify patients that can profit from the therapy. The field of immuno-metabolism has attracted the attention of clinical oncologists, because of the potential use of EM to determine the immune state and the profile of the tumor and better characterize patients. Altogether, changes in the metabolic state of the target cells for the immunotherapy represent a promising, early prognostic marker of treatment efficacy.

The current methods to determine the energetic metabolic profile of cells can be classified in three groups, that all use bulk cell cultures. The first group, uses metabolic inhibitors (i.e. 2-DeoxyGlucose/2-DG and Oligomycin A/Oligo) and monitor changes in extracellular acidification rate, as well as other indirect but precise consequences of the activity of the different EM pathways (Zhang et al., 2012). The second group measures by mass spectrometry the levels of the different intracellular metabolites. The third group,- includes methods that monitor, in fixed cells or lysates, the activity of enzymes implicated in particular metabolic pathways (Miller et al., 2017). With the exception of the method of Miller et al. these techniques all need purified cells in high amount, require extensive manipulations, equipment, and also access to large enough tissue sample, and thus cannot generally be applied to characterize live cells obtained from human patients or biopsies.

Thus, considering the multiple potential applications in biology and medicine but the current insufficient techniques, it still a need to develop a simple and quick method for determining energetic metabolism profile with single cell resolution.

**SUMMARY OF THE INVENTION:**

As defined by the claims, the present invention relates to a method of profiling the energetic metabolism of a population of cells.

As defined by the claims, the present invention also relates to use of the method of the invention for diagnostic purpose, for predicting the survival time of a patient suffering from cancer or for predicting whether a subject suffering from cancer will be eligible to a therapy, in particular chemotherapy or immunotherapy.

As defined by the claim, the present invention also relates to a kit for performing the method of the invention.

**DETAILED DESCRIPTION:**

The inventors designed a method, called here ZENITH, which rapidly and efficiently measures the protein synthesis level in a population of cells upon inhibition of the different energy producing pathways. Indeed the inventors combined puromycin incorporation with a novel anti-puromycin monoclonal antibody to develop a reliable method to perform EM profiling with single cell level resolution based on protein synthesis intensity as read-out. The method developed by the inventors permits to acquire energetic metabolism profiles with **single cell resolution in non-abundant cells ex-vivo** and permits to decrease to a **minimum manipulation time,** incubations and cost of sample preparation. ZENITH, is a novel method to monitor energetic metabolism (EM) activity in individual cells, that can be applied to ex-vivo samples containing complex and heterogeneous cell populations. ZENITH can be performed in few hours without any requirement for extensive purification procedures, dedicated equipment, nor specific technical expertise. ZENITH versatility particularly enlarges the horizon of cellular metabolism studies in human clinical samples and the inventors provide here examples of ZENITH's capability to profile EM variations in human T cells and DC subsets from blood or isolated from tumor beds, including a newly identified and rare AXL+CD22+ DC population. Moreover, by using this functional metabolic profiling of immune cells, the inventors were able to exploit single cell RNA-seq data to identify genes whose pattern of expression highly correlates with different functional EM profile. Based on this EM gene list, the inventors analysed the RNA-seq data of sorted antigen presenting cells (APCs) isolated from 450 human tumors (i.e lung, head and neck, colon-rectal, bladder and hepatic cancers). They observed that APCs of patients fall into two clear clusters, one displaying a respiratory-APC gene expression profile, and other with glycolytic-APC gene expression profile.

Accordingly the first object of the present invention relates to a method of profiling the energetic metabolism profile of a population of cells comprising:

i) providing four samples [S1], [S2], [S3] and [S4] of said population of cells

ii) measuring the protein synthesis level [LCo] in sample [S1] in absence of any inhibitor;

iii) contacting the sample [S2] with an inhibitor [A] of the production of the energy resulting from glycolysis and the oxidative phosphorylation of glucose-derived pyruvate and measuring the protein synthesis level [LA] in said sample;

iv) contacting the sample [S3] with an inhibitor [B] of the production of the energy resulting from TCA cycle and oxidative phosphorylation comprising pyruvate oxidation, oxidation of fatty acids and oxidation of amino acids and measuring the protein synthesis level [LB] in said sample;

v) contacting the sample [S4] cells with both inhibitors [A] and [B] and measuring the protein synthesis level [L(A+B)] in said sample

vi) assessing the glucose dependency of the population of cells; wherein the glucose dependency of the cells is assessed by calculating the formula (I):

$$\text{Glucose dependency} = ([LCo]-[LA]) \: / \: ([LCo]-[L(A+B)]) \: x \: 100 \quad (I)$$

vii) assessing the mitochondrial dependency of the population of cells; wherein the mitochondrial dependency of the cells is assessed by calculating the formula (II):

$$\text{Mitochondrial dependency} = ([LCo]-[LB]) \: / \: ([LCo]-[L(A+B)]) \: x \: 100 \quad (II)$$

viii) assessing the glycolytic capacity of the population of cells; wherein the glycolytic capacity of the cells is assessed by calculating the formula (III):

$$\text{Glycolytic capacity} = (1 - ([LCo]-[LB]) \: / \: ([LCo]-[L(A+B)])) \: x \: 100$$
$$(III)$$

ix) assessing the capacity for oxidation of fatty acids and oxidation of amino acids of the population of cells, , wherein the oxidation of fatty acids and oxidation of amino acids capacity is assessed by calculating the formula (IV):

$$\text{Oxidation of fatty acids \& oxidation of amino acids capacity} = (1 - ([LCo]-[LA]) \: / \: ([LCo]-[L(A+B)])) \times 100 \quad (IV)$$

and

x) finally determining the energetic metabolism profile of the population of cells.

As used herein, the term "cell" refers to any eukaryotic cell. Eukaryotic cells include without limitation ovary cells, epithelial cells, immune cells, hematopoietic cells, bone marrow cells, circulating vascular progenitor cells, cardiac cells, chondrocytes, bone cells, beta cells, hepatocytes, and neurons. Moreover the term includes pluripotent stem cells. As intended herein, the expression "pluripotent stem cells" relates to division-competent cells, which are liable to differentiate in one or more cell types. Preferably, the pluripotent stem cells are not differentiated. Pluripotent stem cells encompass stem cells, in particular adult stem cells (e.g. mesenchymal stem cells (MSC)) and embryonic stem cells. The term also encompasses induced pluripotent stem cells (IPS). Accordingly the term includes purified primary cells and immortalized cell lines. The term also refers to cells in suspension (e.g. circulating leukocytes (PBMC)), or adherent cells (e.g. endothelial cells).

In some embodiments, the population of cells consists of a homogeneous population of cells. As used herein, the term "homogeneous population of cells" refers to a population of cells comprising one cell type and/or one cell state. Typically, said population of cells was previously sorted or isolated by any routine method.

In some embodiments, the population of cells consists of a heterogeneous population of cells. As used herein, the term "heterogeneous population of cells" refers to a population of cells comprising two or more different cell types or cell states.

For instance said population of cells may result from any biological sample obtained from a subject (e.g. a patient). As used herein, the term "biological sample" refers to any sample of biological origin potentially containing a population of cells. Examples of biological samples include tissue, organs, or bodily fluids such as whole blood, plasma, serum, tissue, lavage or any other specimen. In some embodiments, the biological sample is a tissue sample that typically results from a biopsy. In some embodiments, the sample is a tumor tissue sample. The term "tumor tissue sample" means any tissue tumor sample derived from the patient. In some embodiments, the tumor sample may result from the tumor resected from the patient. In some embodiments, the tumor sample may result from a biopsy performed in the primary tumour of the patient or performed in metastatic sample distant from the primary tumor of the patient. In some embodiments, the tumor tissue sample encompasses (i) a global primary tumor (as a whole), (ii) a tissue sample from the centre of the tumor, (iii) lymphoid islets in close proximity with the tumor, (iv) the lymph nodes located at the closest proximity of the tumor, (v) a tumor tissue sample collected prior surgery (for follow-up of patients after treatment for example), and (vi) a distant metastasis. In some embodiments, the tumor tissue sample, encompasses pieces or slices of tissue that have been removed from the tumor, including following a surgical tumor resection or following the collection of a tissue sample for biopsy. The tumor tissue sample can, of course, be subjected to a variety of well-known post-collection preparative and storage techniques. Typically, the cells are dissociated from the tissue in order to prepare a suspension of cells. A common method to obtain suspensions from primary tissue is enzymatic disaggregation.

In some embodiments, the population of cells comprises immune cells. As used herein, the term "immune cell" includes cells that are of haematopoietic origin and that play a role in the immune response. Immune cells include lymphocytes, such as B cells and T cells; natural killer cells; myeloid cells, such as monocytes, neutrophils macrophages, dendritic cells, eosinophils, mast cells, basophils, and granulocytes. In some embodiments, the population of cells comprises T cells. As used herein, the term "T cell," refers to a type of lymphocytes that play an important role in cell-mediated immunity and are distinguished from other lymphocytes, such as B cells, by the presence of a T-cell receptor on the cell surface. Several subsets of T cells have been described and typically include helper T cells (e.g., Th1, Th2, Th9 and Th17 cells), cytotoxic T cells, memory T cells, regulatory/suppressor T cells (Treg cells), natural killer T cells, [gamma/delta] T cells, and/or autoaggressive T cells (e.g., TH40 cells), unless otherwise indicated by context. In some embodiments, the term "T cell" refers specifically to a helper T cell. In some embodiments, the term "T cell" refers more specifically to a TH17 cell (i.e., a T cell that secretes IL-17). In some embodiments, the term "T cell" refers to a regulatory T cell or "Treg" cell.

As used herein, the term "profiling" in the context of the invention means defining the energetic metabolism profile of the population of cells.

As used herein, the term "energetic metabolism" designates all biological pathways and reactions that contribute to creating or stocking energy products or metabolites in a cell.

As used herein, the term "protein" has its general meaning in the art refers to an amino acid chain. The term "protein synthesis" (or translation) refers to the process in which ribosomes in the cytoplasm or endoplasmic reticulum synthesize proteins into the cell.

As used herein, the term "production of energy" refers to any process into the cell resulting on synthesis of energetic molecule such as adenosine triphosphate (ATP) and Guanosine-triphosphate (GTP).

As used herein, the term "glycolysis" has its general meaning in the art and refers to the metabolic oxidation of glucose by cells. During glycolysis, glucose is oxidized to pyruvate. Generally, under aerobic conditions pyruvate is dominantly converted into Acetyl-CoA. When oxygen is depleted, pyruvate is converted to lactate and excreted as dominant product of glycolysis.

As used herein the term "oxidative phosphorylation of pyruvate" has its general meaning in the art and refers to the process into the mitochondria which comprises the electron transport chain that establishes a proton gradient (chemiosmotic potential) across the boundary of inner membrane by oxidizing the NADH produced from the Krebs cycle. ATP is synthesized by the ATP synthase enzyme when the chemiosmotic gradient is used to drive the phosphorylation of ADP. The electrons are finally transferred to exogenous oxygen and, with the addition of two protons, water is formed.

As used herein the term "inhibitor of the production of the energy resulting from glycolysis and oxidative phosphorylation of pyruvate" refers to any compound, natural or synthetic, that blocks, suppress, or inhibits partially or totally the production of the energy resulting from glycolysis and the oxidative phosphorylation of pyruvate. According to the present invention said inhibitor is named inhibitor [A].

In some embodiments, the inhibitor [A] is selected from the group consisting of 2-Deoxy-Glucose, 2-[N-(7-Nitrobenz-2-oxa-1,3-diaxol-4-yl)amino]-2-deoxyglucose/2-NBDG, Phloretin, 3-Bromophyruvic acid, Iodoacetate, Fluoride and 6-Aminonicotinamide.

In some embodiments, step iii) is performed in presence of an amount of pyruvate. As used herein, the term "pyruvate" has its general meaning in the art and refers to the conjugate base CH3COCOO- of pyruvic acid. According to said embodiments, the presence of pyruvate allows measuring the glycolysis dependency (PDH dependent). The term "glycolysis dependency (PDH dependent)" means the capacity of cells to sustain energy production when glycolysis is inhibited (i.e. 2-Deoxy-Glucose) in the presence of pyruvate. In this condition pyruvate is transformed into Acetyl-CoA by PDH.

In some embodiments, step iii) is performed in presence of an amount of acetate. As used herein, the term "acetate" or "ethanoate" has its general meaning in the art and refers to the ion of formula CH3COO-. According to said embodiments, the presence of pyruvate allows measuring the glycolysis dependency (PDH independent). The term "Glycolysis dependency (PDH independent)" means the capacity of cells to sustain energy production when glycolysis is inhibited (i.e. 2-Deoxy-Glucose) in the presence of Acetate. In this condition acetate can be converted into Acetyl-CoA, in dependently from PDH activity.

As used herein, the term "PDH" has its general meaning in the art and means Pyruvate Dehydrogenase, a key regulatory enzyme that convert pyruvate into Acetyl-CoA; linking the product of glycolysis with the Krebs cycle.

As used herein, the term "oxidation of fatty acids" or "beta-oxidation" has its general meaning in the art and refers to the catabolic process by which fatty acid molecules are broken down in the mitochondria in eukaryotes to generate acetyl-CoA, which enters the citric acid cycle, and NADH and FADH2, which are co-enzymes used in the electron transport chain.

As used herein, the term "oxidation of amino acids" has its general meaning in the art and refers to the oxidation of amino acids to produce acetyl-CoA, acetate, pyruvate, alpha-ketoglutarate or other metabolites that can enter the TCA cycle and that results in ATP and/or GTP synthesis.

As used herein, the term "inhibitor of the production of the energy resulting from oxidative phosphorylation comprising oxidation of fatty acids and oxidation of amino acids" refers to any compound, natural or synthetic, that blocks, suppress, or inhibits partially or totally the production of the energy resulting from oxidative phosphorylation comprising oxidation of fatty acids and oxidation of amino acids. According to the present invention said inhibitor is named inhibitor [B].

In some embodiments, the inhibitor [B] is selected from the group consisting of Oligomycin (A/B/C/D/E//F and derivates), Rotenone, Carbonyl cyanide-p-trifluoromethoxyphenylhydrazone/FCCP, Trimetazidine/TMZ, 2[6(4-chlorophenoxy)hexyl]oxirane-2-carboxylate/Etomoxir, Bis-2-(5-phenylacetamido-1,3,4-thiadiazol-2-yl)ethyl sulfide/BPTES, and enasidenib. Other inhibitors of wild type or mutant enzymes of mitochondrial metabolism pathways may also be used. As used herein the term "mutation" has its general meaning in the art and refers to any change in DNA or protein sequence that deviates from wild type mitochondrial enzyme. This includes single base DNA changes, single amino acid changes, multiple base changes in DNA and multiple amino acid changes. This also includes insertions, deletions and truncations of a gene and its corresponding protein. For, instance, inhibitors of the following enzymes may be used: Citrate synthase, NAD+ malate dehydrogenase, NAD+ glutamate dehydrogenase, Succinate cytochrome c reductase, Rotenone-sensitive NADH cytochrome c reductase, Adenylate kinase, Rotenoneinsensitive NADH cytochrome c reductase, Monoamine oxidase, and Kynurenine hydroxylase. In particular, inhibitors of isocitrate dehydrogenase (IDH) may be used. As used herein, the term "IDH" has its general meaning in the art and refers to the isocitrate dehydrogenase. IDH is an enzyme which participates in the citric acid cycle. It catalyzes the third step of the cycle: the oxidative decarboxylation of isocitrate, producing alpha-ketoglutarate (a-ketoglutarate or a-KG) and C02 while converting NAD(P)+ to NAD(P)H. This is a two-step process, which involves oxidation of isocitrate (a secondary alcohol) to oxalosuccinate (a ketone), followed by the decarboxylation of the carboxyl group beta to the ketone, forming alpha-ketoglutarate. Another isoform of the enzyme catalyzes the same reaction; however this reaction is unrelated to the citric acid cycle, is carried out in the cytosol as well as the mitochondrion and peroxisome, and uses NADP+ as a cofactor instead of NAD+. Mutations in IDH1 and IDH2 are well known in the art and typically reside in the active site of the enzyme and participate in isocitrate binding. In many instances, they are missense alterations affecting arginine-140 (R140) residue in the IDH2 protein. IDH1 mutants lack the wild-type enzyme's ability to convert isocitrate to a a-ketoglutarate but gains a neomorphic activity which leads to the conversion of a-KG to the oncometabolite 2- hydroxyglutarate (2HG).

As used herein, the term "glucose dependency" refers to the inability of cell to produce energy without glucose. The glucose dependency of the cells is assessed by calculating the formula (I):

$$\text{Glucose dependency} = ([LCo]-[LA]) / ([LCo]-[L(A+B)]) \times 100 \quad (I)$$

As used herein the term "mitochondrial dependency" refers to the inability of cell to produce energy without energetic mitochondrial pathways. The mitochondrial dependency of the cells is assessed by calculating the formula (II):

$$\text{Mitochondrial dependency} = ([LCo]-[LB]) / ([LCo]-[L(A+B)]) \times 100 \quad (II)$$

As used herein the term "glycolytic capacity" refers to the ability of cells to produce energy when all other pathways, but not glycolysis, are inhibited. The glycolytic capacity of the cells is assessed by calculating the formula (III):

$$\text{Glycolytic capacity} = (1 - ([LCo]-[LB]) / ([LCo]-[L(A+B)])) \times 100 \quad (III)$$

As used herein the term "oxidation of fatty acids and oxidation of amino acids capacity" refers to the ability of cells to produce energy when all other pathways, but not oxidation of fatty acids and oxidation of amino acids, are inhibited. The oxidation of fatty acids and oxidation of amino acids capacity is assessed by calculating the formula (IV):

$$\text{Oxidation of fatty acids \& oxidation of amino acids capacity} = (1 - ([LCo]-[LA]) / ([LCo]-[L(A+B)])) \times 100 \quad (IV)$$

In some embodiments, the cells are contacting with the inhibitor during a short period of time, between 20 and 40 minutes, depending on the cell type. For example 20 minutes inhibition is enough in Fibroblasts, while in human blood T cells 40 minutes is optimal. Incubation for longer time periods (i.e. 2 hours of more) with the inhibitors can lead to undesired effects, such as the induction of genes involved in compensatory mechanisms, induction of cell death or activation of cellular stress pathways that block metabolic activities of the cell.

In some embodiments, the method of the present invention further comprises the steps of:

- providing a further sample [S5] of the population of cells
- contacting said sample with an inhibitor [C] of the production of the energy resulting from oxidation of fatty acids and measuring the protein synthesis level [LC] in said sample and,
- assessing the dependency of oxidation of fatty acids of the population cells, , wherein the dependency of oxidation of fatty acids is assessed by calculating the formula (V):

$$\text{Dependency of oxidation of fatty acids} = ([LCo]-[LC]) / ([LCo]-[L(A+B)]) \times 100 \quad (V).$$

As used herein, the term "inhibitor of the production of the energy resulting from oxidation of fatty acids" refers to any compound, natural or synthetic, that blocks, suppress, or inhibits partially or totally the production of the energy resulting from oxidation of fatty acids. According to the present invention, the inhibitor is named inhibitor [C].

In some embodiments, the inhibitor [C] is selected from the group consisting of Trimetazidine/TMZ and 2[6(4-chlorophenoxy)hexyl]oxirane-2-carboxylate/Etomoxir.

As used herein, the term "dependency of oxidation of fatty acids" refers to the inability of cell to produce energy and sustains metabolic activities when the oxidation of fatty acids is inhibited. The dependency of oxidation of fatty acids is assessed by calculating the formula (V):

$$\text{Dependency of oxidation of fatty acids} = ([LCo]-[LC]) / ([LCo]-[L(A+B)]) \times 100 \quad (V)$$

In some embodiments, the method of the present invention further comprises the steps of:

- providing a further sample [S6] of the population of cells
- contacting the sample with an inhibitor [D] of the production of the energy resulting from oxidation of amino acids and measuring the protein synthesis level [LD] in the sample and
- assessing the dependency of oxidation of amino acids of the population cells, wherein the dependency of oxidation of amino acids is assessed by calculating the formula (VI):

$$\text{Dependency of oxidation of amino acids} = ([LCo]-[LD]) / ([LCo]-[L(A+B)]) \times 100 \quad (VI)$$

As used herein, the term "inhibitor of the production of the energy resulting from oxidation of amino acids" refers to any compound, natural or synthetic, that blocks, suppress, or inhibits partially or totally the production of the energy resulting from oxidation of amino acids. According to the present invention, the inhibitor is named inhibitor [D].

In some embodiments, the inhibitor [D] is selected from the group consisting of Bis-2-(5-phenylacetamido-1,3,4-thiadiazol-2-yl)ethyl sulfide/BPTES, Aminooxyacetic acid/AOA and epigallocatechin-3-gallate/EGCG.

As used herein, the term "dependency of oxidation of amino acids" refers to the inability of cell to produce energy and sustains metabolic activities when the oxidation of amino acids is inhibited. The dependency of oxidation of fatty acids is assessed by calculating the formula (VI):

Dependency of oxidation of amino acids = ([LCo]-[LD]) / ([LCo]-[L(A+B)]) × 100          (VI)

In some embodiments, the measurement of protein synthesis level is determined by any well known in the art. For instance, the protein synthesis level is determined as described in Schmidt, E.K., Clavarino, G., Ceppi, M., and Pierre, P. (2009). SUnSET, a nonradioactive method to monitor protein synthesis. Nat Methods 6, 275-277. Briefly, the method is a nonradioactive fluorescence-activated cell sorting-based assay, which allows the monitoring and quantification of global protein synthesis in individual mammalian cells and in heterogeneous cell populations. More particularly, the method involves the use of monoclonal antibodies to puromycin to directly monitor translation using standard immunochemical methods. Puromycin (puro) is an antibiotic that due to its tRNA-AA mimetic molecular structure is very efficiently incorporated into the nascent polypeptide chains during the process of mRNA translation by the Ribosomes.

Accordingly, in some embodiments, the sample is contacted with an amount of puromycin and then after with an amount of monoclonal antibodies specific for purmoycin that are typically conjugated with a detectable label.

Suitable detectable labels include, for example, a heavy metal, a fluorescent label, a chemiluminescent label, an enzyme label, a bioluminescent label or colloidal gold. Methods of making and detecting such detectably-labeled immunoconjugates are well-known to those of ordinary skill in the art, and are described in more detail below.

In some embodiments, the antibodies are labeled with a fluorescent compound. The presence of a fluorescently-labeled antibody is determined by exposing the immunoconjugate to light of the proper wavelength and detecting the resultant fluorescence. Particular examples of detectable labels include fluorescent molecules (or fluorochromes). Numerous fluorochromes are known to those of skill in the art, and can be selected, for example from Life Technologies (formerly Invitrogen), e.g., see, The Handbook-A Guide to Fluorescent Probes and Labeling Technologies). Examples of particular fluorophores that can be attached (for example, chemically conjugated) to a nucleic acid molecule (such as a uniquely specific binding region) are provided in U.S. Pat. No. 5,866, 366 to Nazarenko et al., such as 4-acetamido-4'-isothiocyanatostilbene-2,2' disulfonic acid, acridine and derivatives such as acridine and acridine isothiocyanate, 5-(2'-aminoethyl) aminonaphthalene-1-sulfonic acid (EDANS), 4-amino -N- [3 vinylsulfonyl)phenyl]naphthalimide-3,5 disulfonate (Lucifer Yellow VS), N-(4-anilino-1-naphthyl)maleimide, antl1ranilamide, Brilliant Yellow, coumarin and derivatives such as coumarin, 7-amino-4-methylcoumarin (AMC, Coumarin 120), 7-amino-4-trifluoromethylcouluarin (Coumarin 151); cyanosine; 4',6-diarninidino-2-phenylindole (DAPI); 5',5"dibromopyrogallol-sulfonephthalein (Bromopyrogallol Red); 7 -diethylamino -3 - (4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulforlic acid; 5-[dimethylamino] naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives such as eosin and eosin isothiocyanate; erythrosin and derivatives such as erythrosin B and erythrosin isothiocyanate; ethidium; fluorescein and derivatives such as 5-carboxyfluorescein (FAM), 5-(4,6dicl1lorotriazin-2-yDarninofluorescein (DTAF), 2'7'dimethoxy-4'5'-dichloro-6-carboxyfluorescein (JOE), fluorescein, fluorescein isothiocyanate (FITC), and QFITC Q(RITC); 2',7'-difluorofluorescein (OREGON GREEN®); fluorescamine; IR144; IR1446; Malachite Green isothiocyanate; 4-methylumbelliferone; ortho cresolphthalein; nitrotyrosine; pararosaniline; Phenol Red; B-phycoerythrin; o-phthaldialdehyde; pyrene and derivatives such as pyrene, pyrene butyrate and succinimidyl 1-pyrene butyrate; Reactive Red 4 (Cibacron Brilliant Red 3B-A); rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, rhodamine green, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl rhodamine; tetramethyl rhodamine isothiocyanate (TRITC); riboflavin; rosolic acid and terbium chelate derivatives. Other suitable fluorophores include thiol-reactive europium chelates which emit at approximately 617 mn (Heyduk and Heyduk, Analyt. Biochem. 248:216-27, 1997; J. Biol. Chem. 274:3315-22, 1999), as well as GFP, LissamineTM, diethylaminocoumarin, fluorescein chlorotriazinyl, naphthofluorescein, 4,7-dichlororhodamine and xanthene (as described in U.S. Pat. No. 5,800,996 to Lee et al.) and derivatives thereof. Other fluorophores known to those skilled in the art can also be used, for example those available from Life Technologies (Invitrogen; Molecular Probes (Eugene, Oreg.)) and including the ALEXA FLUOR® series of dyes (for example, as described in U.S. Pat. Nos. 5,696,157, 6, 130, 101 and 6,716,979), the BODIPY series of dyes (dipyrrometheneboron difluoride dyes, for example as described in U.S. Pat. Nos. 4,774,339, 5,187,288, 5,248,782, 5,274,113, 5,338,854, 5,451,663 and 5,433,896), Cascade Blue (an amine reactive derivative of the sulfonated pyrene described in U.S. Pat. No. 5,132,432) and Marina Blue (U.S. Pat. No. 5,830,912). The conjugation of the label to monoclonal antibodies can be accomplished using standard techniques known to the art. Typical methodology in this regard is described by Kennedy et al., Clin. Chim. Acta 70:1, 1976; Schurs et al., Clin. Chim. Acta 81:1, 1977; Shih et al., Int'l J. Cancer 46:1101, 1990; Stein et al., Cancer Res. 50:1330, 1990; and Coligan, supra.

In some embodiments, methods of flow cytometry are thus suitable methods for detecting and measuring the level labelled anti-puromycin antibodies. Flow cytometry is a wellaccepted tool in research that allows a user to rapidly analyze and sort components in a sample fluid. Flow cytometers use a carrier fluid (e.g., a sheath fluid) to pass the sample components, substantially one at a time, through a zone of illumination. Each sample component is illuminated by a light source, such as a laser, and light scattered by each sample component is detected and analyzed. The sample components can be separated based on their optical and other characteristics as they exit the zone of illumination. Said methods are well known in the art. For example, fluorescence activated cell sorting (FACS) may be therefore used and typically involves using a flow cytometer capable of simultaneous excitation and detection of multiple fluorophores. The cytometric systems may include a cytometric sample fluidic subsystem, as described below. In addition, the cytometric systems include a cytometer fluidically coupled to the cytometric sample fluidic subsystem. Systems of the present disclosure may include a number of additional components, such as data output devices, e.g., monitors, printers, and/or speakers, data input devices, e.g., interface ports, a mouse, a keyboard, etc., fluid handling components, power sources, etc. Preferred methods typically involve the permeabilization of the cells preliminary to flow cytometry. Any convenient means of permeabilizing cells may be used in practicing the methods.

In some embodiments, the antibody be labelled with a metallic chemical element such as lanthanides. Lanthanides offer several advantages over other labels in that they are stable isotopes, there are a large number of them available, up to 100 or more distinct labels, they are relatively stable, and they are highly detectable and easily resolved between detection channels when detected using mass spectrometry. Lanthanide labels also offer a wide dynamic range of detection. Lanthanides exhibit high sensitivity, are insensitive to light and time, and are therefore very flexible and robust and can be utilized in numerous different settings. The lanthanide series of the periodic table comprises 15 elements, 14 of which have stable isotopes (La, Ce, Pr, Nd, Sm, Eu, Gd, Tb, Dy, Ho, Er, Tm, Yb, Lu). They are also referred to as rare earth elements. Lanthanides may be detected using CyTOF technology. CyTOF is inductively coupled plasma time-of-flight mass spectrometry (ICP-MS) (See, Cheung et al., "Screening: CyTOF-the next generation of cell detection," Nature Reviews Rheumatology, 7:502-503, 2011, and Bendall et al., "Single-Cell Mass Cytometry of Differential Immune and Drug Responses Across a Human Hematopoietic Continuum," Science, 332(6030):687-696, 2011. CyTOF instruments that are commercially available (e.g. Fluidigm, California, USA) are capable of analyzing up to 1000 cells per second for as many parameters as there are available stable isotope tags.

In some embodiments, the antibody is conjugated to a DNA barcode is used as a fingerprint for labelling the antibody of interest. Such DNA-barcoded antibodies are suitable to convert detection of proteins into a quantitative, sequenceable readout. The DNA barcode is an oligonucleotide of a predefined sequence. According to the invention, the DNA barcode comprises at least 4 nucleotides. DNA barcoding provides the advantage to allow creating an indefinite number of combinations that will lead to a very high specific detection of the antigen and finally will allow using a plurality of DNA-barcoded antibodies in multiplex assays. For instance the DNA-barcoded antibodies act as synthetic transcripts that are captured during most large-scale oligodT-based scRNA-seq library preparation protocols (e.g. 10x Genomics, Dropseq, ddSeq). The principle of the method that is named CITE-seq is described in Stoeckius, Marlon, et al. "Simultaneous epitope and transcriptome measurement in single cells." Nature methods 14.9 (2017): 865.

In some embodiments, the method of the present invention further comprises identifying a particular cell type in said population of cells, in particular by using of a panel of binding partners specific for some cell surface markers of interest (e.g. BCR, CD19 or CD20 for B cells and TCR, CD4, CD8, CD25 for T cells). The binding partners are thus conjugated to the labels as above described.

In some embodiments, determining the energetic metabolism profile of the cell according step x) of the method of the present invention comprises concluding that said cell presents a respiratory profile or a glycolysis profile. As used herein, the term "respiratory profile" means that the cell uses predominantly the mitochondrial respiration to produce energy. As used herein, the term "glycolysis profile" means that the cell uses predominantly the glycolysis to produce energy.

The method of the present invention is thus suitable for metabolic characterization of a cell population. The TCA cycle (also known as Krebs cycle) is a major metabolic pathway that is thought to be used by quiescent cells. The TCA cycle and oxidative phosphorylation efficiently generate ATP in cells whose primary requirements are energy and longevity. The TCA cycle is a nexus for multiple nutrient inputs. Most notably, glucose-derived pyruvate, fatty acids or amino acids are converted into acetyl coenzyme A (acetyl-CoA) that joins the TCA cycle to form citrate. Three major end products of the TCA cycle are GTP, reduced Nicotin-Adenosine Dinucleotide (NADH/H) and reduced Flavin-Adenosine Dinucleotide (FADH2). The nicotinamide and flavin dinucleotides, transfer electrons to the mitochondrial electron transport chain and support oxidative phosphorylation, ultimately leading to ATP generation. The glycolytic metabolic pathway (also termed glycolysis) begins with glucose uptake from the environment and subsequent intracellular processing in the cytosol to produce ATP, pyruvate and other metabolites. Glycolysis is a relatively inefficient pathway for the generation of cellular ATP, producing only two molecules of ATP per degraded glucose molecule. However, glycolysis allows for the reduction of NAD+ to NADH, which is used as co-factor by numerous enzymes and support anabolic growth. The glycolytic flux is maintained through pyruvate reduction into lactate to recycle NADH and maintain NAD+ levels, leading to acidification of extracellular media. Glycolysis plays therefore an essential role in the metabolism of rapidly proliferating cells responding to different environmental cues.

In some embodiments, the method of the present invention combines at least one further analytical technique. In some embodiments, sequencing is performed. As used herein, the term "sequencing" generally means a process for determining the order of nucleotides in a nucleic acid. A variety of methods for sequencing nucleic acids is well known in the art and can be used. In some embodiments, next generation sequencing is carried out. As used herein, the term "next generation sequencing" has its general meaning in the art and refers to sequencing technologies having increased throughput as compared to traditional Sanger- and capillary electrophoresis-based approaches, for example with the ability to generate hundreds of thousands or millions of relatively short sequence reads at a time. Some examples of next generation sequencing techniques include, but are not limited to, sequencing by synthesis, sequencing by ligation, and sequencing by hybridization. Examples of next generations sequencing methods include pyrosequencing as used by the GS junior and GS FLX Systems (454 Life Sciences), sequencing by synthesis as used by Illumina's Miseq and Solexa system, the SOLiD™ (Sequencing by Oligonucleotide Ligation and Detection) system (Life Technologies inc.), and ion Torrent Sequencing systems such as the Personal Genome Machine or the Proton Sequencer (Life Technologies Inc), and nanopore sequencing systems (Oxford nanopore). In the case of sequencing by synthesis using Illumina's sequencing technology, the source molecule may be PCR amplified before delivery to a flow cell.

The method of the present invention may find various applications.

For instance, the method of the present invention is particular suitable for determining activation state of a cell. Actually, as demonstrated in the EXAMPLES, the activation state of a cell can correlate directly to its metabolism profile. Typically, acute immune activation state correlate with a glycolytic profile. As used herein, the term "immune activation state" is based, for example, in the demonstrated capacity of different innate and adaptive immune cells (i.e. T cells, CAR T, B cells, NK, DC and others) to be stimulated by the recognition of particular molecules (i.e Antigens, PAMPs, DAMPs). Activated cells change their metabolic profile, and thus the metabolic profile can be a functional measure of their state of activation.

In some embodiments, the method of the present invention is particular suitable for diagnostic purposes.

In some embodiments, the method of the present invention is particular suitable for diagnosing inflammatory diseases. The term "inflammatory disease" has its general meaning in the art and refers to any disease and condition associated with inflammation. The term may include, but is not limited to, (1) inflammatory or allergic diseases such as systemic anaphylaxis or hypersensitivity responses, drug allergies, insect sting allergies; inflammatory bowel diseases, such as Crohn disease, ulcerative colitis, ileitis and enteritis; vaginitis; psoriasis and inflammatory dermatoses such as dermatitis, eczema, atopic dermatitis, allergic contact dermatitis, urticaria; vasculitis; spondyloarthropathies; scleroderma; respiratory allergic diseases such as asthma, allergic rhinitis, hypersensitivity lung diseases, and the like, (2) autoimmune diseases, such as arthritis (rheumatoid and psoriatic), osteoarthritis, multiple sclerosis, systemic lupus erythematosus, diabetes mellitus, glomerulonephritis, and the like, (3) graft rejection (including allograft rejection and graft-v- host disease), and (4) other diseases in which undesired inflammatory responses are to be inhibited (e. g., atherosclerosis, myositis, inflammatory CNS disorders such as stroke and closed-head injuries, neurodegenerative diseases, Alzheimer's disease, encephalitis, meningitis, osteoporosis, gout, hepatitis, nephritis, sepsis, sarcoidosis, conjunctivitis, otitis, chronic obstructive pulmonary disease, sinusitis and Bechet's syndrome).

In some embodiments, the method of the present invention is particularly suitable for predicting the survival time of a patient suffering from cancer. The method of the present invention is particularly suitable for predicting the duration of the overall survival (OS), progression-free survival (PFS) and/or the disease-free survival (DFS) of the cancer patient. Those of skill in the art will recognize that OS survival time is generally based on and expressed as the percentage of people who survive a certain type of cancer for a specific amount of time. Cancer statistics often use an overall five-year survival rate. In general, OS rates do not specify whether cancer survivors are still undergoing treatment at five years or if they've become cancer-free (achieved remission). DSF gives more specific information and is the number of people with a particular cancer who achieve remission. Also, progression-free survival (PFS) rates (the number of people who still have cancer, but their disease does not progress) includes people who may have had some success with treatment, but the cancer has not disappeared completely. As used herein, the expression "short survival time" indicates that the patient will have a survival time that will be lower than the median (or mean) observed in the general population of patients suffering from said cancer. When the patient will have a short survival time, it is meant that the patient will have a "poor prognosis". Inversely, the expression "long survival time" indicates that the patient will have a survival time that will be higher than the median (or mean) observed in the general population of patients suffering from said cancer. When the patient will have a long survival time, it is meant that the patient will have a "good prognosis".

As used herein, the term "cancer" has its general meaning in the art and refers to a group of diseases involving abnormal cell growth with the potential to invade or spread to other parts of the body. The term "cancer" further encompasses both primary and metastatic cancers. Examples of cancers that may treated by methods and compositions of the invention include, but are not limited to, circulating tumor cells, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. **In** addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; non encapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extramammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brennertumor, malignant; phyllodestumor, malignant; synovial sarcoma; mesothelioma, malignant; dysgerminoma; embryonal carcinoma; teratoma, malignant; strumaovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblasticodontosarcoma; ameloblastoma, malignant; ameloblasticfibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocyticleukemia; mast cell leukemia; megakaryoblasticleukemia; myeloid sarcoma; and hairy cell leukemia.

It has been recently shown that the metabolic state of circulating tumor cells determines their capacity to generate metastasis or to stay in a latent state.

Accordingly, in some embodiments, the method of the present invention is particularly suitable for predicting whether a subject suffering from cancer will develop metastasis.

As used herein, the term "metastasis" or "metastatic cancer" is well-known in the art and refers to the spread of the cancer at another organ. A metastatic tumor is formed at a location distant from the primary lesion as a result of the metastasis of the primary tumor. This is one of the most important concerns in cancer therapy. Specifically, even if a primary lesion is treated, a patient may die because of the growth of a tumor that has metastasized to another organ.

In some embodiments, the method of the present invention is particularly suitable for predicting whether a subject suffering from cancer will be eligible to a therapy.

In other word, the method of the present invention is particularly suitable for predicting the therapy response of a subject suffering from cancer.

For instance, the therapy is chemotherapy. As used herein, the term "chemotherapy" has its general meaning in the art and refers to the treatment that consists in administering to the patient a chemotherapeutic agent. Chemotherapeutic agents include, but are not limited to alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g. , calicheamicin, especially calicheamicin gammall and calicheamicin omegall ; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholinodoxorubicin, 2-pyrrolino-doxorubicin and deoxy doxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; antiadrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxaliplatin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-1 1); topoisomerase inhibitor RFS 2000; difluoromethylomithine (DMFO); retinoids such as retinoic acid; capecitabine; and pharmaceutically acceptable salts, acids or derivatives of any of the above.

In some embodiments, the method of the present invention is particular suitable for prediction and prognosis of therapy efficacy.

In some embodiments, the therapy is immunotherapy. As used herein, the term "immunotherapy" refers to a therapy for a disease that relies on an immune response. In some embodiments, the immunotherapy is selected from the group consisting of checkpoint inhibitors, chimeric antigen receptors (CAR) T cells, Bi-specific T-cell engagers (BiTEs) and adoptive cell therapy. The term "CAR T cells therapy" refers to a therapy consisting using T lymphocytes expressing chimeric antigen receptor. In some embodiments, the immunotherapy involve use of an immune checkpoint inhibitor. The term "immune checkpoint inhibitor", as used herein, refers to a substance that blocks the activity of molecules involved in attenuating the immune response. Examples of immune checkpoint inhibitor includes PD-1 antagonist, PD-L1 antagonist, PD-L2 antagonist CTLA-4 antagonist, VISTA antagonist, TIM-3 antagonist, LAG-3 antagonist, IDO antagonist, KIR2D antagonist, A2AR antagonist, B7-H3 antagonist, B7-H4 antagonist, and BTLA antagonist. The term "Bi-specific T-cell engagers" (BiTEs) refers to a therapy consisting of artificial bispecific monoclonal antibodies that are investigated for the use as anti-cancer drugs. BiTEs, target the host's immune system, more specifically the T cells' cytotoxic activity, against cancer cells. BiTEs form a link between T cells and tumor cells and are being tested in clinical trials.

As used herein, the term "predicting" refers to a probability or likelihood for a patient to respond to the treatment with an immunotherapy or chemotherapeutic agent. As used herein, the term "responsiveness" refers to ability to assess the likelihood that treatment will or will not be clinically effective.

A further object of the present invention relates to a kit or a reagent for practicing the method of the present invention. The subject reagents and kits thereof may vary greatly. However, the kit of the present invention comprises an inhibitor [A] of the production of the energy resulting from glycolysis and the oxidative phosphorylation of glucose-derived pyruvate and an inhibitor [B] of the production of the energy resulting from TCA cycle and oxidative phosphorylation comprising pyruvate oxidation, oxidation of fatty acids and oxidation of amino acids as well as puromycin and monoclonal antibodies specific to puromycin. The kit of the present invention may further comprises inhibitor [C] of the production of the energy resulting from oxidation of fatty acids and inhibitor [D] of the production of the energy resulting from oxidation of amino acids. In some embodiments, the kit of the present invention may comprise an amount of pyruvate and/or acetate. In some embodiments, the kit of the present invention comprise reagents employed in the various methods, such as panel of antibodies for cell sorting. The kit may also comprise various buffer mediums. The kits can further include a software package for calculating the different formulas (I-VI) suitable for assessing the metabolic profile (i.e. different dependencies). In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, on which the information has been recorded. Yet another means that may be present is a website address which may be used via the internet to access the information at a removed, site. Any convenient means may be present in the kits. The above-described analytical methods may be embodied as a program of instructions executable by computer to perform the different aspects of the invention. Any of the techniques described above may be performed by means of software components loaded into a computer or other information appliance or digital device. When so enabled, the computer, appliance or device may then perform the above-described techniques to assist the analysis of sets of values associated with a plurality of genes in the manner described above, or for comparing such associated values. The software component may be loaded from a fixed media or accessed through a communication medium such as the internet or other type of computer network. The above features are embodied in one or more computer programs may be performed by one or more computers running such programs. Software products (or components) may be tangibly embodied in a machine-readable medium, and comprise instructions operable to cause one or more data processing apparatus to perform the different calculations as described above. The computer may also displaying the results by any relevant presentation (e.g. chart, histograms, Also provided herein are software products (or components) tangibly embodied in a machine-readable medium, and that comprise instructions operable to cause one or more data processing apparatus to perform operations comprising: storing sequence data for a multitude of sequence reads. In some examples, a software product (or component) includes instructions for assigning the sequence data into V, D, J, C, VJ, VDJ, VJC, VDJC, or VJ/VDJ lineage usage classes or instructions for displaying an analysis output in a multi-dimensional plot. In some cases, a multidimensional plot enumerates all possible values for one of the following: V, D, J, or C. (e.g., a three-dimensional plot that includes one axis that enumerates all possible V values, a second axis that enumerates all possible D values, and a third axis that enumerates all possible J values). In some cases, a software product (or component) includes instructions for identifying one or more unique patterns from a single sample correlated to a condition. The software product (or component) may also include instructions for normalizing for amplification bias. In some examples, the software product (or component) may include instructions for using control data to normalize for sequencing errors or for using a clustering process to reduce sequencing errors. A software product (or component) may also include instructions for using two separate primer sets or a PCR filter to reduce sequencing errors.

The disclosure will be further illustrated by the following figures and examples. However, these examples and figures should not be interpreted as according to the invention and in any way as limiting the scope of the present disclosure.

FIGURES:

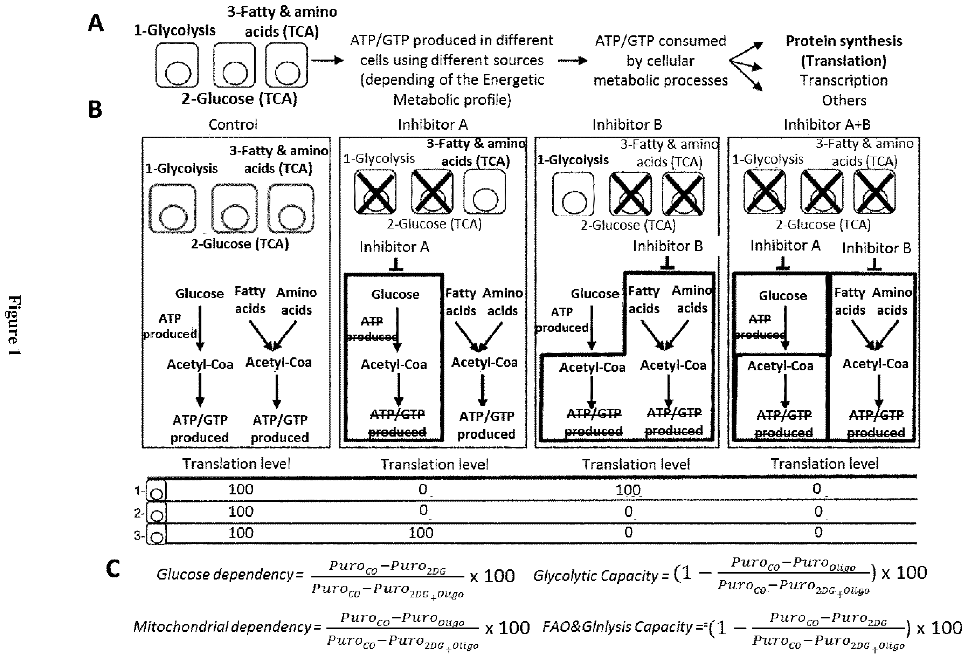

**Figure 1. Rational and principle of ZENITH.** A) Different cells use different source of energy to produce ATP and GTP. These sources are mostly glucose, aminoacids or fatty acids. Cells using these different molecules display different energetic metabolism profiles that are depicted. Protein synthesis consumes around 30% of the cellular energy (ATP/GTP) available and is thus rapidly impacted by nutrients starvation. B) To determine the EM profile using ZENITH, cell samples are subdivided and incubated with or without energetic pathways inhibitors (e.g. No inhibitor, inhibitor A, inhibitor B and inhibitors A+B). Cells produce ATP using glycolysis by oxidative phosphorylation of the glycolysis products (GlycOxPhos) or by degrading Fatty acids and aminoacids (FAO&Glnlysis). Inhibitor A blocks Glycolysis and GlycOxPhos, while inhibitor B blocks the production of energy from GlycOxphos, FAO and amino acids, and the combination of Inhibitors A and B (and or C) blocks the production of ATP from all sources. The bulk of treated cells (Control, A, B and A+B) are processed for flow cytometry with same combination of monoclonal antibodies to identify single cells and protein synthesis level after a short puromycin pulse. The impact of each treatment (energetic pathway inhibitors) on protein synthesis levels is quantified by multiparametric flow cytometry and processed with appropriate analysis sofwares. C) EM profiles are directly inferred from puromycin incorporation levels (MFI puro) in control cells (PuroCo), substracted form the levels found in the cells treated with the different inhibitors as described in the formulas.

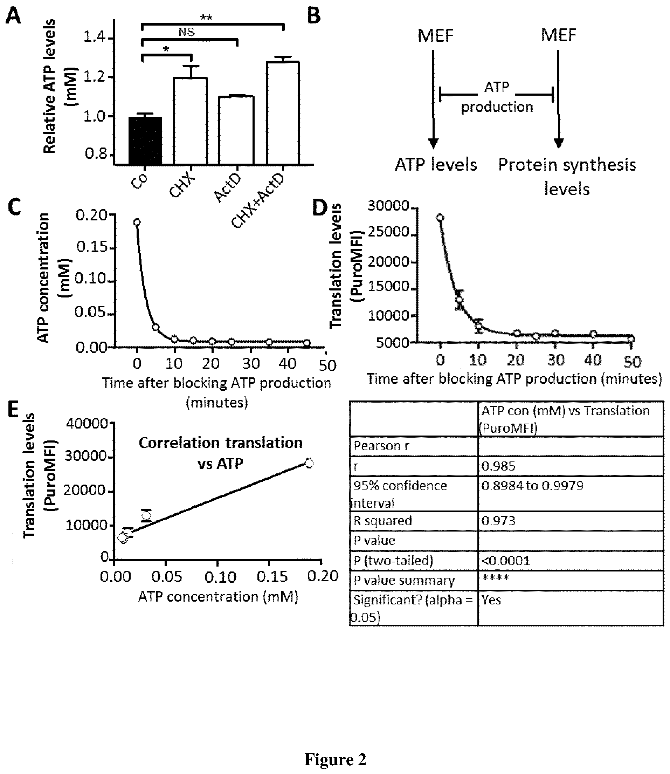

**Figure 2. ATP levels and protein synthesis.** $4 \times 10^4$ MEFs were seeded in 96 well plates and treated with 2-DG+Oligo for different periods of time. A) Effect of protein synthesis and RNA synthesis inhibition on the pool of ATP. Total ATP levels in the absence (Co) or presence of translation inhibitor (CHX), transcription inhibitor (ActD) or both CHX+ActD (5 minutes of treatment). B) Levels of ATP as a function of time after 2-DG+Oligo treatment. C) Levels of translation (Puromycin MFI) as a function of time after 2-DG+Oligo treatment measured in single cells by FACS. D) Correlation between the level of ATP and level of translation (Puro MFI). Two tailed t-test (A) on at least 3 independent experiments (* $p<0.05$; ** $p<0.001$). N= 3 independent experiments (* $p<0.05$; ** $p<0.001$).

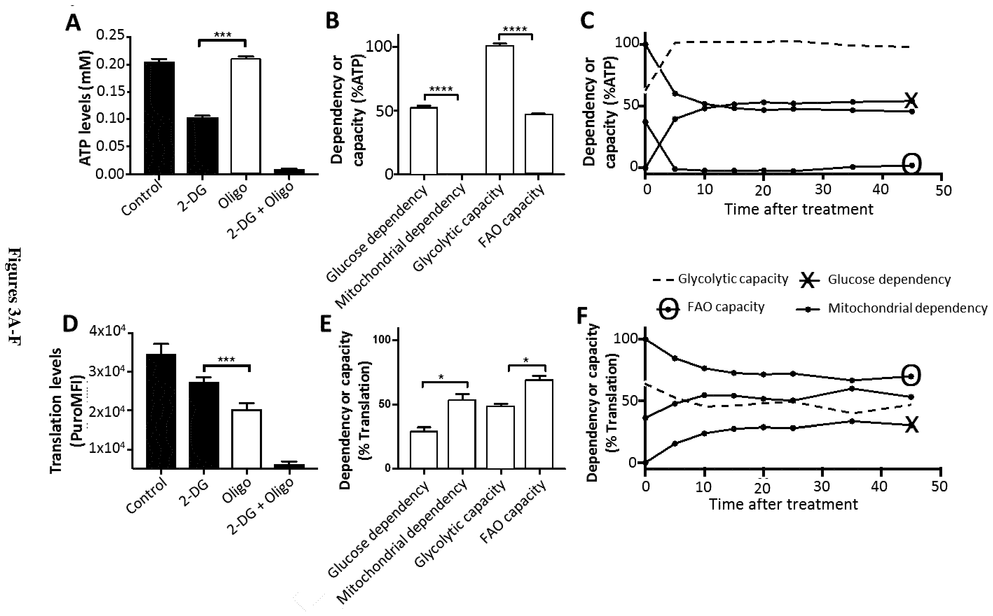

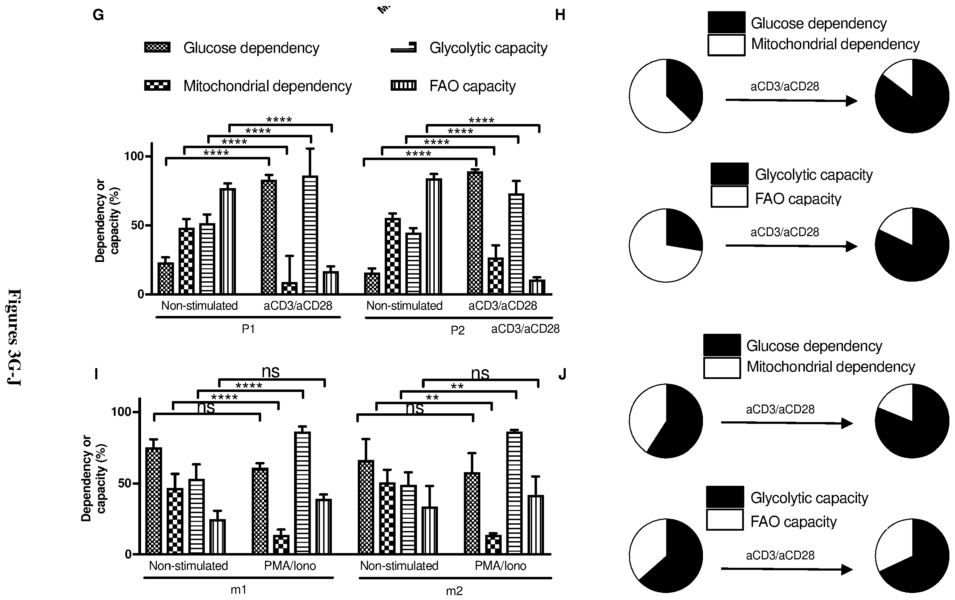

**Figure 3. EM profile using ATP levels or protein synthesis activity in MEFs and changes in EM profile in human and mouse T cells upon activation.** A) ATP levels in MEFs treated for 20-30 minutes with the metabolic inhibitors. B) EM profile established with the ATP levels shown in (A). C) EM profile established with ATP levels at different timepoints after adition of metabolic inhibitors. D) Level of protein synthesis in MEFs treated for 20 minutes with the metabolic inhibitors. E) EM profile calculated with the Puro incorporation levels shown in (D). G) Mouse splenic CD8 T cells where analyzed using ZENITH. Total Puro MFI is indicated in Control cells (non-activated) or PMA/Ionomycin-treated. H) EM profiles established with values shown in (G). I) EM profile of human blood central memory CD4+ T cells from two different subjects (P1 and P3) was generated in control (non-stimulated) or activated (aCD3/aCD28 beads) using the ZENITH method. A combination of antibodies that allow for the identification of central memory CD4 T (CD3+CD4+CD45RO+CCR7+) cells was used to estimate the metabolic profile in the absence or presence of T cells stimulation. N=3, ANOVA on at least 2 independent experiments (* $p<0.05$; ** $p<0.005$; ***$p<0.0005$).

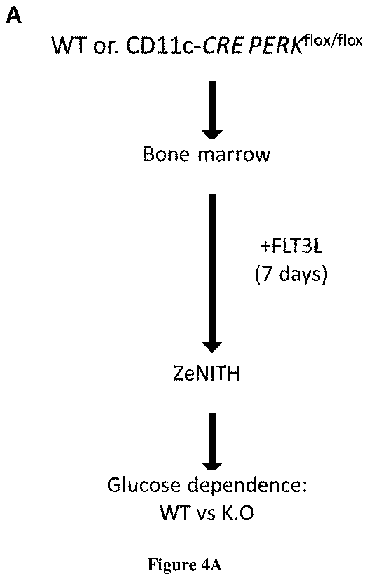

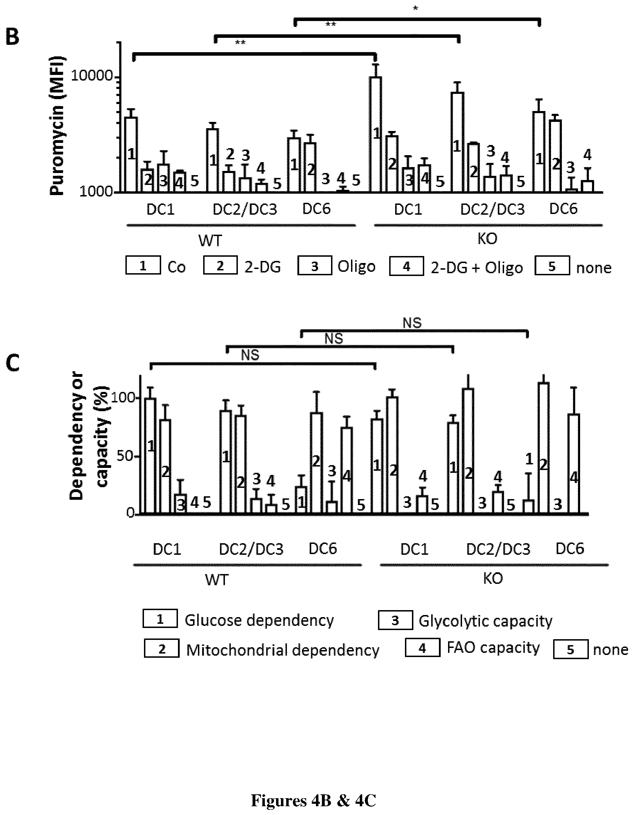

**Figure 4. EM profile based on ZENITH in WT and in PERK KO bmDCs differentiated in vitro with Flt3L.** A) Flt3L-bmDC cultures from WT and CD11c-Cre PERKflox/flox mice were analyzed using ZENITH to determine if PERK and ER-stress are implicated in the translation loss induced by 2-DG treatment. Distinct DC subsets displaying key markers (see methods) are indicated as DC1 (XCR1+), DC2(CD11B+)/DC3 and DC6 (pDC). B) Puromycin incorporation levels measured by flow cytometry in DC subsets from the same in vitro culture. DCs were incubated with Control, 2-DG, Oligo and 2-DG+Oligo prior puromycin incorporation. C) Glucose and mitochondria Dependency or Glycolytic and FAO&Glnlysis (FAO) Capacity, established from the raw values in B) show that even in the absence of PERK, cells undergo translation inhibition. Data shown as mean $\pm$ SD of three independent mice and are representative of two independent experiments; * $p<0.05$, ** $p<0.001$.

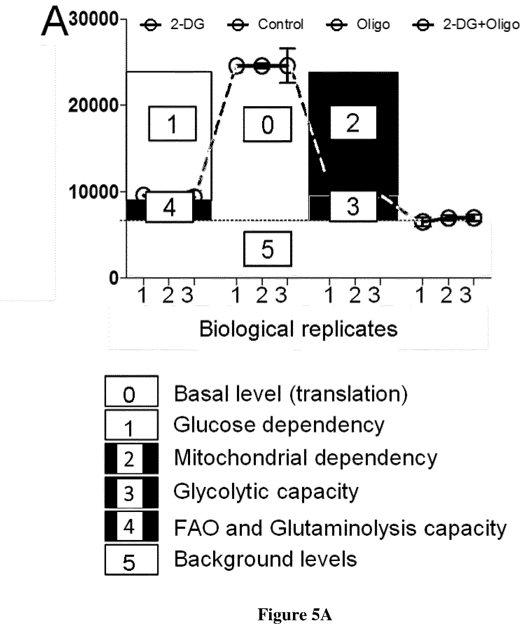

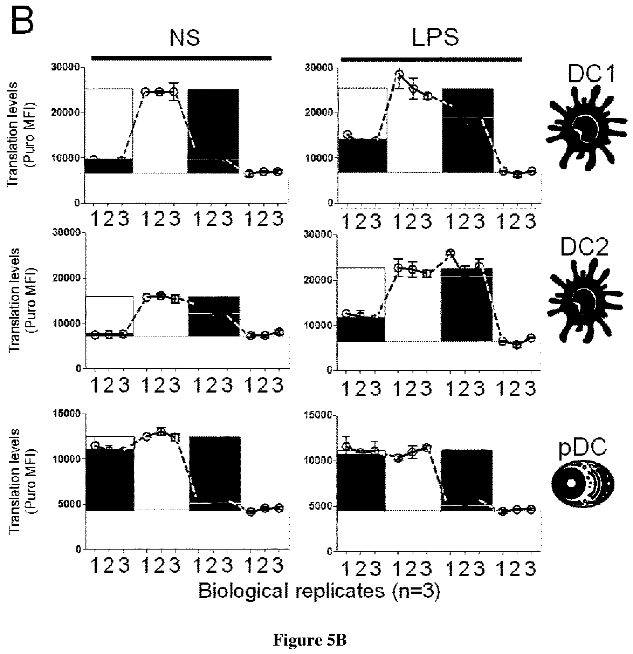

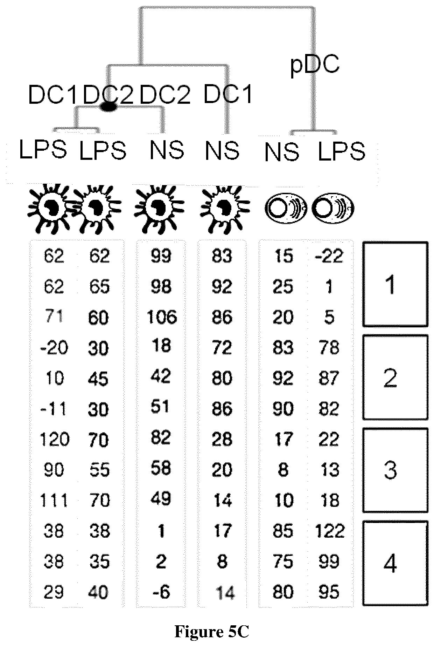

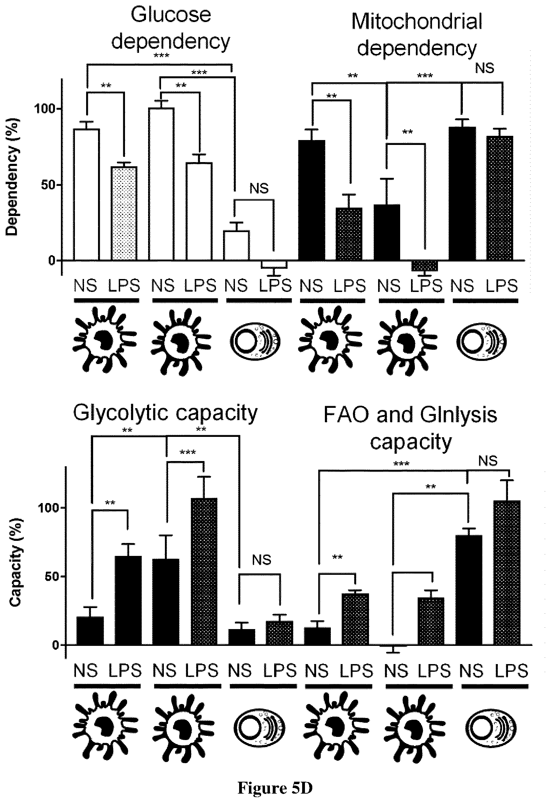

**Figure 5. EM profile based on ZENITH of bone Marrow derived Dendritic cells subsets (BMDCs).** A) Example of protein synthesis profile of BMDCs from three mice (biological replicates) in response to 2DG, Control treatment, Oligomycin and 2-DG+Oligomycin. The area under the curve of protein synthesis levels represent the capacity of the cells to sustain metabolic functions (i.e. Translation) in the presence of each inhibitor, allowing to obtain the metabolic profile (0, 1, 2, 3, 4, 5). B) ZENITH metabolic profile of FLT3L derived BMDCs from three (I, II, III) WT mice non-stimulated (Left) or stimulated during 4 hours with LPS (Right). Dendritic cells subpopulations were analysed and their metabolic profile is shown. C) The metabolic profile of each replicate (n=3) from each mice (n=3), obtained in the different DCs subpopulations can be used to cluster cells subsets with similar metabolic profiles. D) Statistical analysis of the metabolism profile obtained in the different DC subsets, reveals that DC2 are the subset that performs a stronger metabolic switch upon LPS, while pDCs do not change their metabolism.

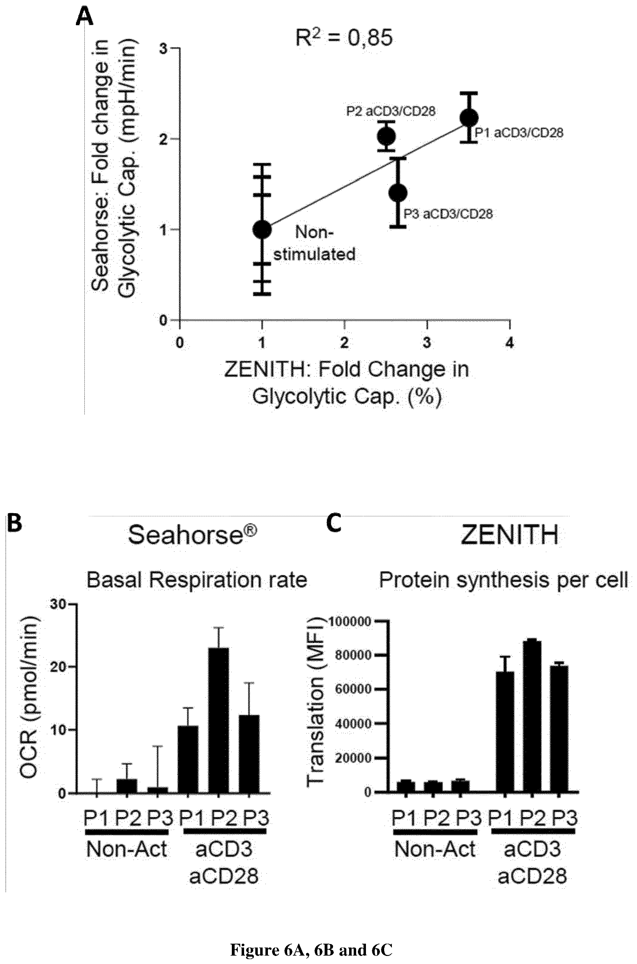

**Figure 6. Parallel Seahorse® and ZENITH metabolic anlaysis of resting and activated T cells.** A) Correlation between the changes in glycolytic capacity of steady state and activated (aCD3/CD28) T cells from three different subjects measured by Seahorse® and ZENITH (P1, P2, P3, Pearson r=0.92; $R^2$=0.85; p<0.01). B) Basal Oxygen Consumption Rate (OCR) in non-activated (non-Act) and activated T cells (aCD3/CD28). Each bar represents the mean of P1, P2 and P3 (in triplicates). C) Basal translation levels (anti-Puro, Geometric Mean Fluorescence, intensity) in non-activated (non-Act) and activated T cells (aCD3/CD28). Each bar represents the mean of P1, P2, and P3 (in triplicates).

**EXAMPLE:**

**Material & Methods**

**Cells and Cell Culture**

Mouse splenocytes from WT C57BL/6J (Jackson) or PERK KO C57BL/6J background mice (Zhang et al., 2002) were cultured in DMEM containing 5% of Fetal Calf Serum (FCS) and 50 $\mu$M of 2-Mercaptoethanol (Mouse cells culture media, MCCM) at 37 °C 5% of CO2. GM-CSF BM-derived dendritic cells (GM-bmDCs) were differentiated in vitro from the bone marrow of 6-8-week-old male mice, using GM-CSF, produced by J558L cells. Bone marrow progenitors were plated at $0.8\times106$ cells/ml, 5 ml/well in 6-well plates, and cultivated with RPMI (GIBCO), 5% FCS (Sigma-Aldrich), 20 $\mu$g/ml gentamycin (Sigma-Aldrich), 50 $\mu$M $\beta$-mercaptoethanol (VWR), and GM-CSF. The medium was replaced every 2 days; BM-derived DCs were used for experiments at day 6. Similarly, FLT3L BM-derived dendritic cells (FLT3L-bmDCs) were differentiated by adding FLT3L to RPMI, 10% of Fetal Calf Serum (FCS) and 50 $\mu$M of 2-Mercaptoethanol (Mouse cells culture media, MCCM) during 6 days at 37 °C 5% of CO2. To obtain splenocytes, eight weeks old wild type C57BL/6J mice were sacrificed by cervical dislocation and splenectomized. Single cells suspentions from the spleens were generated and cultured in MCCM as previously described. Mononuclear cell enriched from blood of healthy donors was submitted to Ficoll-paque plus (PBL Biomedical Laboratories). PBMCs and Whole blood were cultured in the absence (non stimulated) or in the presence of for indicated time. Immune cell stimulations were performed in the absence (Control) or presence of 0,1 $\mu$g/ml of extrapure Lipopolysacharide (Invivogen LPS, Cat. tlrl-3pelps), 10 $\mu$g/ml Poly I:C (Invivogen, Cat. No. tlrl-pic), CpG-A ODN 2216 (Invivogen, Cat. No. tlrl-2216) or PMA (5 ng/ml; Sigma, Cat. no. P-8139) and ionomycin (500 ng/ml; Sigma, cat. no. I-0634) over night for T cell stimulations and 4 hours for Dendritic cells.

## ATP measurement

20x104 MEFs were seeded in 100ul of 5% FCS DMEM culture media ON in opaque 96 well plates. Cells were incubated with the inhibitors for the times indicated in the figures. After, 100ul of Cell titer-Glo luminiscence ATP reconstituted buffer and substrate (Promega, Cat. No. G7570) was added to each well and Luminiscence was measured after 10 minutes following manufacturer instructions. A standard curve with ATP was performed using the same kit and following manufacturer instructions.

## Metabolic flux analysis (Seahorse®)

OCR and ECAR were measured with the XF24 Extracellular Flux Analyzer (Seahorse Bioscience). 4.105 cells with $\alpha$CD3/$\alpha$CD28 beads or not, were placed in triplicates in XF medium (nonbuffered Dulbecco's modified Eagle's medium containing 2.5 mM glucose, 2 mM L-glutamine, and 1 mM sodium pyruvate) and monitored 25 min under basal conditions and in response to 10mM Glucose, 1 $\mu$M oligomycin, 100 mM 2-Deoxy-Glucose. Glycolytic capacity was measured by the difference between ECAR level after add oligomycin and before add glucose. OCR, ECAR and SRC parameters was analyzed and extract from Agilent Seahorse Wave Desktop software. Glycolytic capacity was obtained by the difference between ECAR level after add Oligomycin and before add Glucose.

## ZENITH

Cells were plated at 1-10×106 cells/ml, 0,5 ml/well in 48-well plates. Experimental duplicates were performed in all conditions. After differentiation, activation or harvesting of human of cells, wells were treated during 45 minutes with Control, 2-DeoxyGlucose (2-DG, final concentration 100mM; Sigma-Aldrich Cat. No. D6134-5G), Oligomycin (Oligo, final concentration 1$\mu$M; Sigma-Aldrich Cat. No.75351), BPTES (final concentration 1$\mu$M; Cat. No. SML0601), Trimetazidine (TMZ, final concentration 1$\mu$M; Sigma-Aldrich Cat. No. 653322) or a combination of the drugs at the final concentrations before mentioned. As negative control, the translation initiation inhibitor Harringtonine was added 15 minutes before addition of Puromycin (Harringtonine, 2 $\mu$g/ml; Abcam, cat. ab141941). Puromycin (Puro, final concentration 10 $\mu$g/ml; Sigma-Aldrich, Cat. No. P7255) is added during the last 15 minutes of the metabolic inhibitors treatment. After puro treatment, cells were washed in cold PBS and stained with a combination of fluorochrome cell viability marker and conjugated antibodies against different surface markers during 30 minutes at 4°C in PBS 5% FCS, 2mM EDTA (FACS wash buffer). After washing with FACS wash buffer, cells were fixed and permeabilized using BD Cytofix/Cytoperm™ (Catalog No. 554714) following manufacturer instructions. Intracellular staining of Puro using fluorescently labeled anti-Puro monoclonal antibodies was performed by incubating cells during one hour (1:1000, Clone 12D10, Merk, Catalog No. MABE343) at 4°C diluted in Permwash.

## Flow cytometry

Flow cytometry was conducted using BD LSR II and BD LSR Fortessa X-20 machine (BD Biosciences™) and data were analyzed with FlowJo (Tree Star™) and/or Cytobank. The antibodies used to stain mouse splenocytes were anti-Puro-AF488 (Merk, Catalog No. MABE343) or anti-Puro-Clone R4743L-E8, rat IgG2A in house produced and conjugated with Alexa Fluor 647 or Alexa-Fluor 488, anti-Phospho-S6-PE (Cell Signaling Technology, Catalog No. #5316), anti-Ki67 PE-eFluor 610 (eBioscience™, Catalog No. 61-5698-82) CD4-APC-eF780 (eBioscience™, Catalog No. 47-0042-82), CD8-APC (eBioscience™, Catalog No. 17-0081-83), CD80-PercPCy5.5 (Biolegend™, Catalog No. 104722), anti-B220-BV421 (Biolegend™, Catalog No. 103251), anti-MHC-II-AF700 (eBioscience™, Catalog No. 56-5321-82), LIVE/DEAD™ Fixable Aqua Dead Cell Stain (Invitrogen™, Catalog No. L34957). The following anti-Human antigens antibodies were used for staining whole blood and PBMCs upon ZENITH protocol application. Alexa Fluor-488 Mouse Anti-Human Axl (Clone 108724, R&D Biosystems, Cat. No. FAB154G), Alexa Fluor-647 Mouse Anti-Puromycin (clone 12D10, Millipore, Cat. No. MABE343-AF647), BUV395 Mouse Anti-Human CD11c (Clone B-ly6, BD Bioscience, Cat. No. 563787), BUV737 Mouse Anti-Human CD86 (Clone FUN-1, BD Bioscience, Cat. No. 564428), BV510 Mouse Anti-Human CD19 (Clone HIB19, BD Bioscience, Cat. No. 740164), BV510 Mouse Anti-Human CD3 (Clone HIT3a, BD Bioscience, Cat. No. 564713), BV510 Mouse Anti-Human CD56 (Clone B159, BD Bioscience, Cat. No. 740171), BV605 Anti-Human HLA-DR (Clone L243, BioLegend, Cat. No. BLE307640), BV650 Mouse Anti-Human CD16 (Clone 3G8, BD Bioscience, Cat. No. 563692), BV711 Mouse Anti-Human CD14 (Clone M5E2, BD bioscience, Cat. No. 740773), BV785 Mouse Anti-Human CD45RA (Clone HI100, BioLegend, Cat. No. BLE304140), Live Dead Fixable Aqua Dead Cell Stain Kit (Life Technologies, Cat. No. L34957), PE Rabbit anti-Phospho-S6 Ribosomal Protein (Ser235/236) monoclonal (Clone D57.2.2E, Cell signaling, Cat. No. 5316S), PE Rat Anti-Human Clec9A/CD370 (Clone 3A4, BD Bioscience, Cat. No. 563488), PE-Cy7 Mouse Anti-Human CD22 (Clone HIB22, BD Bioscience, Cat. No. 563941), AF488 Mouse Anti-Human CD38 (Clone HIT-2, BioLegend, Cat. No. BLE303512).

**Animal studies**

Wild type C57BL/6 mice were purchased from Charles River and maintained in the animal facility of CIML under specific pathogen-free conditions. C57Bl/6 embryos at gestation embryonic day 12.5 (E12.5) were depleted of all organs and brain, and dissociated in Liberase Tm (0.5mg/ml, Roche), DNaseI (0.2mg/ml, Roche) in PBS for 15min at 37° C while stirring constantly. Cell suspensions were washed with RPMI (Thermo scientific), supplemented with 2% heat-inactivated FCS, 100U/ml penicillin, and 100 mg/ml streptomycin prior Immunostaining and flow cytometry analysis.

This study was carried out in strict accordance with the recommendations in the Guide for the Care and Use of Laboratory Animals the French Ministry of Agriculture and of the European Union. Animals were housed in the CIML animal facilities accredited by the French Ministry of Agriculture to perform experiments on alive mice. All animal experiments were approved by Direction Départementale des Services Vétérinaires des Bouches du Rhône (Approval number A13-543). All efforts were made to minimize animal suffering.

**Statistical analysis**

Statistical analysis was performed with GraphPad Prism software. When several conditions were to compare, we performed a one-way ANOVA, followed by Tukey range test to assess the significance among pairs of conditions. When only two conditions were to test, we performed Student's t-test or Welch t-test, according the validity of homoscedasticity hypothesis (* $P<0.05$, ** $P<0.01$, *** $P<0.005$).

Results

Given the hurdles find in other metabolism analytical techniques, our objective was to develop a method responding to three main imperatives: A) To profile EM in non-abundant cells ex-vivo; B) To decrease to a minimum manipulation time, incubations and cost of sample preparation; C) To acquire EM profiles with single cell resolution. Changes in the EM profile require the activation of different signaling pathways and also genetic reprograming that enable cells to change their catabolism. ZENITH is based on this concept and integrates that most of the energy that the cell obtain from degrading glucose, aminoacids or lipids is constantly consumed by the protein synthesis machinery (Buttgereit and Brand, 1995; Lindqvist et al., 2017; Schimmel, 1993). Conceptualizing that protein synthesis intensity directly reflects ATP consumption, we designed a method that rapidly and efficiently measure by flow cytometry protein synthesis activity in single cells upon inhibition of the different energy producing pathways (**Fig. 1**). The two inhibitors that are shown in the illustrations and used in the experiments are 2-Deoxy-D-Glucose (2-DG, Inhibitor A) and Olygomycin A (Oligo, inhibitor B). Glucose can be used in two ways to produce ATP, Glycolysis alone (**Fig 1B**) or Glycolysis followed by Oxidative Phosphorylation (GlycOxPhos). As shown in **figure 1B,** the presence of inhibitor A, blocks ATP/GTP production from glucose degradation, and only allow cells to produce ATP/GTP from acetyl-CoA derived from oxidation of fatty acids (FAO) or oxidation of amino acids ( ie Glnlysis). In contrast, the inhibitor B, impairs the ATP production from mitochondrial respiration (OxPhos), also resulting in the inhibition of the TCA cycle, and consequently inhibiting energy production from acetyl-CoA, FAO and Glnlysis, and thus cells can only produce ATP to sustain protein synthesis by performing Glycolysis. Cells that have a glycolytic EM profile, express a set of enzymes with nucleoside di-phosphate kinase activity (NDPK/NME) that rapidly exchange ATP to GTP and are able to sustain translation when mitochondrial respiration is inhibited. In contrast, cells that have a respiratory EM profile, that are forced to switch to glycolysis (i.e. by blocking mitochondrial respiration) can potentially produce ATP to survive, but they are not adapted to generate GTP and do not sustain protein synthesis efficiently.

Classically, measurements of the rate of protein synthesis required the use of radioactive amino acids. Puromycin (puro) is an antibiotic, that due to its tRNA-AA mimetic molecular structure is very efficiently incorporated into the nascent polypeptide chains during the process of mRNA translation by the Ribosomes. We have previously developed and patented fluorescent monoclonal antibodies anti-puromycin and demonstrated that the level of anti-puromycin staining is a very precise measure of the level of protein synthesis (Schmidt et al., 2009), patent FR08 56499). To further optimize the signal to noise ratio of puromycin intracellular detection during ZENITH, we screened and developed a novel monoclonal anti-puromycin antibody specifically adapted to intracellular flow cytometry (**data not shown**). The metabolic profile of each cell is obtained by the combination of a panel of monoclonal antibodies that enable us to identify single cells from different types, and a directly coupled to fluorochrome anti-Puro monoclonal antibody to monitor the level of protein synthesis by the use of multiparametric flow cytometry. Taking into account the mechanism of action of each of the inhibitors, the direct interactions between the EM pathways and global protein synthesis intensity, we apply a mathematic formula to integrate the results obtained from the different measurements into one simple graph that illustrates the metabolic profile of each cell subsets (**Fig. 1C**). The capacity of certain cells to compensate and change their metabolism upon treatment with inhibitors, can be misinterpreted as a defect of our method. ZENITH measure the glycolytic capacity of cells when mitochondrial respiration is inhibited, and the glucose dependency in the presence of 2-DG. The FAO and Glnlysis dependency can also be calculated in the presence of TMZ (FAO inhibitor) and BPTES (Glutaminolysis inhibitor). Moreover, the low dependency in any of the sources can be used to identify the cells with high degree of EM plasticity. In other words, we quantify the degree of synergy in the presence of inhibitors of several pathways, as compared to the sum of effects of the inhibitors separately. This measure allow us to identify cells with a more plastic and rapidly adaptable EM metabolism, something that is informative and was only observed in certain cells.

**Changes in ATP levels tightly correlate with protein synthesis activity.**

Our methodology was validated by correlating total ATP levels with protein synthesis measured by flow cytometry. Using non-transfromed mouse embryonic fibroblasts (MEFs), we tested whether translation or transcription inhibition (with Cycloheximide/CHX, or ActinomycinD/ActD, respectively) impacted ATP levels, when the different energetic pathways are inhibited (Oligo+2-DG). As shown in **Figure 2A**, only when CHX was added, the pool of ATP was found to be significantly higher compared to Oligo+2-DG treatment (Co) (**Fig. 2A**). This result, is in accordance with previous results showing that translation has a higher energetic cost as compared to transcription, and thus is a more sensitive marker of the metabolic status of the cells. After, to determine if they show similar pattern we tested how the levels of ATP and the level of protein synthesis change as function of time upon blockade of ATP synthesis. We incubated MEFs during different times with Oligo+2-DG, and ATP levels were quantified by luminescence using Cell titer-Glo® (**Fig. 2B**), and protein synthesis after puromycin incorporation by flow cytometry (**Fig. 2C**). We observed a statistically significant correlation between the levels of ATP and the level of total protein synthesis (**Fig. 2D**, Pearson r=0,985, R squared=0,9703, p<0.0001). These results demonstrate that variation in intracellular levels of ATP and of protein synthesis activity can be directly correlated and that translation intensity can be used as a read out to monitor variations in ATP availibility and synthesis and perform EM profiling.

**Protein synthesis levels in response to metabolic inhibitors describe better the EM activity than bulk ATP levels.**

Human and mouse embryonic fibroblasts have been shown to display high Oxygen Consumption Rate (OCR) and low ExtraCellular Acidification Rate (ECAR) (Suganya et al., 2015; Zhang et al., 2012). This important ratio of OCR/ECAR is characteristic of nontransformed cells that have high mitochondrial activity, relative to a low glycolytic activity (Suganya et al., 2015; Zhang et al., 2012). As a consequence MEFs do not acidify their culture media, even when cultured at high density. This phenotype is also observed in resting T cells, but is rapidly lost when T cells are activated by APCs and engage in a rapid switch to glycolysis (MacIver et al., 2013).

Based on these observations and high mitochondrial dependency of fibroblast and T cells, we compared EM profiles obtained from quantifying ATP levels (bulk ATP levels) to profiles obtained from quantifying protein synthesis by flow. The formulas used for calculating the EM profiles with ATP is developed as such:

$$Glucose\ dependency = \frac{ATP_{Co} - ATP_{2DG}}{ATP_{Co} - ATP_{2DG+Oligo}} x100$$

$$Mitochondrial\ dependency = \frac{ATP_{Co} - ATP_{Oligo}}{ATP_{Co} - ATP_{2DG+Oligo}} x100$$

$$Glycolytic\ Capacity = (1 - \frac{ATP_{Co} - ATP_{Oligo}}{ATP_{Co} - ATP_{2DG+Oligo}}) x100$$

$$FAO\&Glnlysis\ Capacity = (1 - \frac{ATP_{Co} - ATP_{2DG}}{ATP_{Co} - ATP_{2DG+Oligo}}) x100$$

In MEFs the maximum glycolytic capacity calculated by using ATP levels (**Fig. 3A**) is almost 100% and is significantly higher than the maximun FAO&Glnlysis capacity (**Fig. 3B**, p<0.001 legend). By performing kinetic studies, we could observe that the total EM profile remain unchanged, despite being calculated at different times after treatment with the inhibitors (**Fig. 3C**), as exemplified by the levels of ATP that remained unchanged in the presence of Oligomycin (**Fig. 3A and 3B**). These results demonstrate that in highly respiratory cells, like MEFs, global ATP levels after treatment with metabolic inhibitors are not able to reflect energetic mitochondrial dependency. Oligomycin inhibition on respiration could only be observed when 2-DG+Oligo was also added to the cells. These results, evidenced that a measure of ATP levels upon inhibition of the different energy producing pathways does not permit to obtain an accurate EM profile of the cells, due to the induction of probable compensation mechanisms. In contrast, when ZENITH was used to profile the same MEFs, a completely different, but coherent, picture emerged (Fig. 3D, 3E and 3F). ZENITH revealed that MEFs have higher mitochondrial dependency than glucose dependency, and also a higher FAO&Glnlysis capacity than glycolytic capacity (**Fig. 3E**, p<0,05 legend), confirming previous published observation on the EM of these cells (Suganya et al., 2015; Zhang et al., 2012). To further demonstrate the validity of ZENITH, we analyzed mouse splenic cells (n=5l) and CD8+ T cells activated or not for 6 h with PMA/Ionomycin. CD8+ T cells activation leads to a dramatic switch in EM profile, increasing metabolic activity and switching from respiration to aerobic glycolysis (MacIver et al., 2013). Resting splenic CD8 T cells displayed significantly lower metabolic activity than their activated counterparts (**Fig. 3G**, p<0,0001). Determination of the EM transition profile of non-activated to activated CD8 T cells, indicated a strong reduction in mitochondrial energetic dependency (from 55% to less than 15%, **Fig. 3H,** p<0,0001) and an increase in glycolytic capacity, reaching almost 90% (**Fig. 3H**, p<0,0001). These results were confirmed in human central memory CD4+ T cells isolated from blood and after activation with beads coated with the agonistic anti-CD3 and anti-CD28 antibodies (**Fig. 3I**). Althogether these results, demonstrate that ZENITH by analyzing protein synthesis in single cells exposed to metabolic inhibitors provides an accurate measure of the respective contributions of EM pathways activity in individual cells present in heterogenous populations.

**Protein synthesis inhibition upon short exposure to 2-DG is not due to ER-stress induction.**

It has been previously shown that treatment of cells with 2-DG during several hours can induce endoplasmic reticulum (ER) stress due to a defect in glycosylation and folding of recently translated proteins (Liu et al., 2016; Marquez et al., 2017; Zhang et al., 2015). ER-stress induce the activation of PERK, a kinase that phosphorylates the alpha subunit of eukaryotic translation initiation factor 2 (eIF2$\alpha$) that acts as a dominant negative inhibitor of translation initiation. To address whether ER stress could interfere with protein synthesis upon 2-DG treatment, we took advantage of the CD11c-CRE PERKflox/flox mice that delete PERK in all DC subsets (CD11c+ cells, **data not shown**). 2-DG effect was compared both in WT PERKflox/flox (control) and in CD11c-CRE PERKflox/flox (PERK KO) DCs derived from bone marrow precursors with Flt3L. Irrespective of their genetic background, the different DC subsets had the same capacity to decrease protein synthesis in response to 2-DG and displayed the same EM characteristics, suggesting that PERK is not implicated in the loss of translation observed upon the short 2-DG treatment required to perform ZENITH.

**Metabolic profile of *in-vitro* generated and *ex-vivo* mouse Dendritic cells**

Most studies about the metabolism of mouse DCs, have been performed using in-vitro differentiation cultures of bone marrow that enable to obtain large amounts of this cells. DC differentiation in response to FLT3L and GM-CSF, require in-vitro incubation of the hematopoietic precursors during at least one week in fully supplemented media and FCS, prior potential flow based sorting or magnetic purification. Both of these processes may lead to metabolic alterations and major differences with their ex-vivo counterparts. Although the validity of GM-CSF bmDC, as a truly relevant DC model is a matter of debate (Guilliams and Malissen, 2015; Helft et al., 2015; Helft et al., 2016; Lutz et al., 2016), most metabolic studies have been performed using this system (Everts et al., 2012; Kelly and O'Neill, 2015; Wolf et al., 2016). GM-CSF derived bone marrow cultures generate an heterogeneous population of macrophages (GM-Macs), Dendritic cells (GM bmDCs) and immature DCs (Helft et al., 2015; Lutz et al., 2017). The single cell resolution of ZENITH allows the characterization of the metabolic profile of cell populations present in low proportion in the sample, without the need for further manipulation and isolation. We confirmed that the bulk of immature GM-CSF-bmDC show high dependency on glucose and switch towards increased glycolytic capacity and decreased respiration upon activation with LPS **(data not shown),** as previously described (Everts et al., 2012). Upon LPS treatment, not all cells follow exactly in the same kinetics of activation, something that is revealed by their heterogenous levels of maturation markers. While some already start to show a mature phenotype, they also start to show higher dependency on mitochondrial respiration **(data not shown,** LPS immatureDC 5% vs Mature DC-A 45%, P<0.001). Interestingly, this data is in accordance with previous studies suggesting that immature GM-bmDCs have an EM profile that is equivalent to the highly glycolytic human blood monocytes **(data not shown)** (Cheng et al., 2014; Oren et al., 1963).

Moreover, we could identify in the culture, a subpopulation of steady state "mature" DCs charcterized by high levels of surface MHC-II and CD86 (Mature DC-B, **data not shown),** that displayed a distinct metabolic profile and performed different metabolic change after LPS stimulation **(data not shown)**. Although the origin and function of this population remain to be established, it could not have been identified using existing technologies, further highlighting the power of ZENITH to establish EM profiles in an unbiased fashion and concomitantly on different cell subsets. ZENITH allows therefore a functional cell population classification based on the clustering of individual cell metabolic profiles, independently of their abundance in the studied sample.

The EM profile of FLT3L-derived and splenic DC ex-vivo was also established. In contrast to GM-CSF-derived, FLT3L-bmDC encompass several DC subsets including, DC1 (XCR1+ cDC), DC2 (SIRPA+CD11b+ cDC) and DC6 (CD123+ SiglecH+ pDCs) (Merad et al., 2013). FLT3L-derived bmDC were compared to freshly isolated splenic DCs and correlation of EM profiles with the different DC subsets and their activation state was carriedout. Flt3L bmDCs and splenic DCs are highly dependent on mitochondrial respiration, with lower glycolytic capacity in steady state condidions, with DC6/pDC being the most dependent **(data not shown).** In splenic DC, the LPS dependent switch to glycolysis was only partially observed in DC1 and not in DC2 nor DC6. This situation was inverted in Flt3L-bmDC, with DC2 implementing glycolysis almost exclusively, while DC6 remained unaffected. These results could be also dependent on the capacity of the different DC subsets to be activated directly by LPS, with TLR4 being mostly expressed on DC2. Altogether, our results indicate that while the metabolic profile of GM-CSF and FLT3L-bmDCs, do not entirely recapitulate the EM profile of explanted splenic DCs. Upon LPS-activation, a switch to glycolysis can be observed although in different individual subsets according to the origin of the DCs under examination. Nevertheless, EM profiles of mouse DCs strongly correlated with the level of immune activation, but not maturation, and may represent a surrogant marker of inflammatory status.

**ZENITH® recapitulates Seahorse® EM profiling of steady state and activated T cells.**

The metabolic switch of T cells to aerobic glycolysis upon activation was originally documented in the 1970s and more recently confirmed using the Seahorse® method. To benchmark our method, we monitored the variations in EM observed in isolated bulk human blood T cells at steady state or upon activation in parallel by Seahorse® and ZENITH®. Upon activation, an increase in the glycolytic capacity of T cells was observed by both Seahorse® and ZENITH® (**Data not shown**). Measures obtained with the two methods were in excellent agreement (correlation Spearman r squared 0.85, P<0.01) (**Fig. 6A**). We observed a significant decrease in the spare respiratory capacity in bulk of T cells upon activation with Seahorse (Data not shown). Interestingly, an increase in OCR by Seahorse, was paralleled with a strong increase in the global level of PS measured by ZENITH® although at a larger extent (**Fig. 6B and 6C**, respectively). Overall, the EM profiles of T cells upon activation obtained by Seahorse® and by ZENITH® were therefore very consistent, during which the level of translation (**Fig. 6C**) correlated with the global metabolic activity of the cells and changes in the response to inhibitors confirmed the metabolic switch towards aerobic glycolysis that occurs upon T cell activation. However, ZENITH® showed two main advantages over Seahorse® measurements. First, the magnitude of the signal and deviation of measurements with ZENITH® were superior (**Fig. 6B vs 6C**). Second, ZENITH® analysis was performed with 10 fold less T cells ($1,2.10^5$ in triplicates vs. $1,2.10^6$ cells, respectively). Moreover, ZENITH® could incorporate a full FCS spectrum of T cell markers in the analysis allowing to study in parallel different CD3+ T cells subpopulations present into the bulk sample (**Fig. 6**), encompassing naive, memory and effector CD4+ or CD8+ T cells subsets.

**Metabolic deconvolution of blood T cell subsets by ZENITH® identifies a memory CD8+ T cells subset constitutively displaying high glycolytic capacity.**

To expand upon the ability to deconvolve T cell subpopulations, we next applied ZENITH® to mixed populations that previously were inaccessible to single-cell analyses. For this, we took advantage of staining for CD45RA, IL7RA (CD127), CCR7, CD45RO, CD57, PD1, and Perforin all of which allow subset analyses of naïve and memory CD4+ and CD8+ cells from total human blood preparations. Application of antibodies to these nine markers yielded six phenotypically distinct clusters/subpopulations with different abundances (Data not shown). The metabolic profiles of non-activated naive T cells (CD8 or CD4), as well as memory (EM and CM) CD4 and highly differentiated CD8 (HDE) showed a medium-high degree of mitochondrial dependence (**Data not shown**), consistenly to what was previously reported on their metabolic activity[4]. In contrast, the less abundant cell subsets such as CD8 early effector memory (EEM) and Natural Killer (NK) cells (that co-purfied with T cells and represented just 5% of the cells) showed higher glycolytic capacity. To determine if similar metabolic trends are observed in other species and preparations, we performed ZENITH® on resting and activated mouse splenic T cells and human blood central memory CD4 T cell subsets (**Data not shown**). As a result we could also determine a highly consistent switch of mouse and human T cells upon activation towards high glycolytic capacity and high glucose dependence (**Data not shown**).

We note that during bulk T cells analysis, naive cells represented 42%, (the majority out of 78%) and thus likely drives the EM monitoring performed for unsegregated population by Seahorse® (**Fig. 6B**). Thus, Seahorse® measurements indicate a rather low "mean" glycolytic rate/capacity and high "mean" oxygen consumption rate (**Data not shown**). Seahorse analysis of resting T cells is in accordance with the metabolism of naive T cells determined by ZENITH® (**Data not shown**). However, Seahorse results completely masked the presence of CD8+ EEM that represents no more that 5% of the cells (representing 500 cells in each of the treatments, resulting in 2000 cells) and presented, at steady state high glycolytic capacity.

Another important feature of the multiparametric ZENITH® resolutive power is the possibility to feature single cell behaviors according to their sensitivity to metabolic inhibitors independently of their phenotype. This allows to identify functional metabolic heterogeneity first, and to determine the phenotype or sort, different cells afterwards. As a proof of concept, resting purified T cells were treated with Oligomycin to inhibit mitochondrial respiration prior translation monitoring. As result, the histogram plotting translation levels showed two T cell subpopulations, one with high and one with low levels of translation (**Data not shown**). The population that showed high level of translation upon mitochondrial inhibition were labeled as "Glycolytic" and the cells that blocked translation as "Mitochondrial dependent" (**Data not shown**). As shown in the T-SNE, the phenotype of Glycolytic and Respiratory T cells, recapitulated our previous results (**Data not shown**) and showed that the expression of CD45RA, mostly present in naive T and NK cells, correlates well "Mitochondrial dependence" (**Data not shown**). In conclusion, we found that ZENITH® allow for both the measurement of the EM profile of known non-abundant cell subsets of interest, but also to sort and identify "unknown" cells with interesting metabolic profile present within a heterogenous sample.

**Profiling the metabolic state of human tumor-associated myeloid cells.**

Immunotherapies are a game changer in oncology yet only a fraction of patients show complete immune-mediated rejection of the tumor. The variations observed in patients responses to treatment have created a strong need for understanding the functional state of tumor-associated immune cells (immunoprofiling)[6]. We thus tested whether ZENITH® could be used for paralleled phenotypic and metabolic profiling of human tumor samples and what this would reveal about the heterogeneity of immune cell subsets comparing tumors of diverse origins, notably comparing a tumor with tumor-free adjacent tissue. We thus performed ZENITH® using PMBCs from healthy donors, using two cancers from the same tissue (explanted meningioma, brain metastasis (originated from a breast cancer)), and comparing renal carcinoma tumors and renal juxtatumoral tissue. In the case of renal carcinoma and juxtatumoral tissue, both ZENITH® and single cell RNA seq analysis were performed in parallel on the same sample. While we focused on the tissue settings, in our results heatmap, we included the EM profile of when the same immune cells where identified in the blood from healthy donors.

We observed 8 different myeloid populations in meningioma and 6 different subset in renal carcinoma (**Data not shown**), that were all profiled by ZENITH® (**Data not shown**). Upon clustering of the different cell subsets based on EM profiling, two groups emerged, a "Glycolytic cluster" and a "Respiratory cluster" (**Data not shown**). Mono1 and Neutrophils displayed glycolytic metabolism profiles in all blood samples and tumors tested (**Data not shown**). In contrast, Mono2, DC1 and DC2 showed relatively high glycolytic capacity when isolated from kidney tumor and juxtatumoral tissues, while these subsets showed high respiratory metabolism profile in the two brain tumors. Conversely, tumor-associated macrophages (TAM), showed high mitochondrial dependence, while juxta-tumoral macrophages displayed high glycolytic capacity (**Data not shown**), suggesting that tumor microenvironment modifies TAM EM. The decrease of glycolytic capacity in TAM as compared to juxta-tumoral macrophages was previously associated with increased immunosuppression in the tumor environment, tumor progression via both nutritional and immunological cues, and poor patient survival[32]. These results demonstrate again the analytical capacity and descriminative power of ZENITH® and suggest that in addition to the tumor type, it's anatomical origin could influence the metabolism of immune subsets introducing and additional layer of heterogeneity in the tumor environment. This sensitivity would be unknown using currently available bulk measures.

**Linking scRNA-seq and functional energetic metabolism profile in tumor-associated myeloid cells.**

As these results were not previously observed and the ZENITH® method is new, we also sought to extend and validate the findings, by processing in parallel the same sample using single-cell RNA-seq. We therefore aimed to compare in each population, the functional EM profile obtained by ZENITH® and metabolic gene expression profile obtained by mRNA sequencing. To do this, we first identified specific glyolytic and respiratory gene signature that correlate in with functional metabolism in different myeloid cells in the blood (Data not shown). Then we aimed to tested the expression (mRNA levels) of these glycolytic and respiratory metabolic gene signatures in the different myeloid populations of the tumor. To do so, sorted myeloid cells (CD45+Lin-HLA-DR+) from the renal carcinoma and its juxtatumoral tissue and performed single cell RNA-seq using the 10X Genomics Chromium platform paired with deep sequencing (Data not shown). Analysis of 12,801 cells for the tumor and 2,080 for the juxta tumoral tissue yielded 6 and 5 high quality population clusters respectively. To rigorously identify the myeloid populations we checked the expression of characteristic signatures of these populations33 to establish cellular identities in the tSNE representations. This process allowed us to identify both Mono1, Mono2, and DC clusters (**Data not shown**). We focused on 5 monocytes and macrophages (clusters expressing MAFB and/or CSF1R) that we monitored by flow cytometry and were present both in tumor and juxta-tumoral tissue **(Data not shown)**. By checking the expression of classical markers (i.e FCGR3A/CD16 and CD14 **(Data not shown)** we confirmed that clusters 0 and 1 represent CD14+CD16- classical monocytes. Cluster 2 represents CD14-CD16+ non classical monocytes (Mono2), while co-expression of CD14 and CD16 for the clusters 3 and 4, sugest macrophage-like phenotype. Altogether, those results indicate that tumor micro environment specifically modify macrophages metabolism profile functionally and at the transcriptional level. Therefore, single cell RNA sequencing analysis confirmed results obtained by performing ZENITH[®] on all the different myeloid cell subsets identified **(Data not shown)**. Moreover, in strong agreement with our ZENITH[®] data by FACS, monocytes clusters (0,1,2) presented an enrichment in glycolytic signature both in tumor and juxta tumoral tissue. However, and still in agreement with ZENITH[®] functional data, macrophages (cluster 3) showed high expression of the respiratory signature in the tumor while this was not detectable in juxta tumoral tissue **(Data not shown)**. As observed for the monocytes, dendritic cells presented an enrichment in glycolytic signature both in tumor and juxta tumoral tissue **(Data not shown).** Moreover, performing ZENITH[®] allowed us to identify a functional gene signature identified on myeloid cells sorted from PBMC (Data not shown) that can be extended to myeloid cell sorted from tissue and tumors. Therefore, combinig ZENITH[®] profiling and single cell RNA sequencing can be used to profile energetic metabolism of a variety of cell type and tissues

## Discussion

ZENITH represents as a novel and rapid technique to evaluate the energetic metabolism profile of cells by flow cytometry at single cell resolution. We showed that this simple profiling method allows a direct integration of energetic metabolism measure with transcriptomic data, in turn permitting immune cell population clustering, analysis of immunostimulatory activity and immunomonitoring in cancer patients. We could show that while human blood monocytes and neutrophiles exhibit a very strong glucose dependency and glycolytic capacity at steady state, dendritic cells have low glycolytic capacity and higher mitochondrial respiratory activity. Upon LPS activation, DCs subsets, undergo a strong switch towards glycolysis and as Dendritic cells mature an increase in their mitochondrial dependency and decrease their glycolytic capacity is observed.

Altogether, the different glycolytic EM profile observed among blood cells might reflect functional requirements, like for migrating neutrophils, monocytes (Mono1), and moDCs that need to access peripheral tissues that are poorly oxygenized, as compared to the blood stream. EM activity is clearly dependent on a gene expression program that also dictates functional cellular differentiation and is implemented in the cells prior reaching the tissue where they display their effector functions. EM profiling can therefore be used to predict the immune status in patients or organs. Steady state human blood and tonsil DCs or spleen derived DCs migrate to highly irrigated secondary lymphoid tissues, a situation that correlates with their high mitochondrial dependency. Once in secondary lymphoid organs, mature DCs can activate naive and central memory T cells that circulate and remain most of their life between the blood and lymph stream. We have confirmed here that, while naive and central memory T cells show at steady state high mitochondrial dependency (more than 50%) and low glycolytic capacity (less than 45%), their EM profile changes dramatically upon activation, showing a decrease in mitochondrial dependency (to less than 20%) and an increase in glycolytic capacity (to more than 80%). When T cells are activated in the lymph nodes, they proliferate, differentiate in highly glycolytic effector T cells and re-enter in the blood circulation with the ability to find inflammed postcapillary venules and extravasate. However, the first metabolic switch occurs even 6 hours after activation, and thus suggest that even in secondary lymphoid organs, such as the spleen there are zones or situations where high glycolytic potential is required or associated with efficient immune response.

ZENITH will enable researchers to harness the high throughput power of multiparametric flow cytometry that is used for diagnostics in many fields of human medicine (oncology, immunology, infectology, etc), without the need for additional specialized instruments and trained staff to be implemented. The single cell resolution of ZENITH allows for the design a screening strategy based on gene inactivation or silencing to identified genes or cells affecting Energetic matabolism. For example CAS9 based knock out screenings could be used to identify new genes involved in the regulation of the metabolic profile in dendritic cells. The capacity to sort single cells that display a particular metabolic profile and perform next generation sequencing, will for the first time allow the identification of novel genes involved in metabolism and its regulation ex-vivo in a cell type specific manner.

This method can also be used to analyze samples by Flow cytometry but also, to perform ex-vivo imaging of live tissue slices or whole organs by fluorescent microscopy. This will allow us to combine our current knowledge of anatomopathology, with information about metabolism in different areas and cellular activities in the compromised tissue. Ultimately this method has the potential to become a routine pathology study that can bring important information for clinicians with the need of choosing the best treatment against cancer and other diseases.

We plan to perform ex-vivo ZENITH to perform imaging of live tissue slices or whole embryo, organs and tumors by fluorescent microscopy. We will apply this technology to study transgenic mice that express reporter fluorescent protein in immune cells subpopulation that cannot be studied by current available techniques because their attachment to the tissue and membrane composition make it difficult to isolate as intact single cell suspentions.

The field of immunometabolism in oncology has very promising applications. This technology will enable to determine if the metabolic profile of tumor cells and immune cells infiltrating the tumor is informative to predict tumor progression. Transcriptomic data obtained from several tumor sets suggest that the expression pattern of genes involved in tumor metabolism, is a better predictor of tumor progression that the level or kind of immune cells infiltrating the tumor. Thus, ZENITH has the potential to become a method used for diagnostics and immunomonitoring in human oncology, an area of human medicine in most urgent need for novel therapies and markers.

ZENITH has the potential to be applied in many basic and translational research studies, especially in the interphase between oncology, immunology and immunometabolism. It can be used to analyze blood cells, secondary lymphoid organs and tumors and tumor-infiltrating cells and in neurobiology. This method can be used not only to analyze samples by Flow cytometry but also, to perform ex-vivo imaging of live tissue slices or whole organs by fluorescent microscopy. Ultimately, it has the potential to become a routine pathology study that can bring important information for clinicians with the need of choosing the best treatment against cancer and other diseases. Indeed flow cytometers are available in most research institutes and hospitals, ZENITH represents the most accessible method for functional metabolic profiling and presents several advantages regarding to sensitivity, accesibility, single cell resolution, stability of the readout, time required, compatibility with fixation and sorting compared to other methods. Importantly, ZENITH can establish the metabolic profile of very infrequent cells, as exemplified by the analysis of early effector T cells that represent around 5% of the total T cells isolated from blood (500 cells), thus representing a gain of sensitivity of aproximatively 800 fold compared to Seahorse® measurements (400.000 in triplicates).

Given the direct relationship between EM and the functionality of lymphoid effector cells and myeloid cells, ZENITH analysis could be used to define the 'immune EM contexture' and complement the establishment of an immunoscore that's define immune fitness of tumours and predict and stratify patients for tailored therapies.

**REFERENCES:**

Throughout this application, various references describe the state of the art to which this disclosure pertains.

Assmann, N., and Finlay, D.K. (2016). Metabolic regulation of immune responses: therapeutic opportunities. J Clin Invest 126, 2031-2039.

Buttgereit, F., and Brand, M.D. (1995). A hierarchy of ATP-consuming processes in mammalian cells. Biochem J 312 (Pt 1), 163-167.

Cheng, S.C., Quintin, J., Cramer, R.A., Shepardson, K.M., Saeed, S., Kumar, V., Giamarellos-Bourboulis, E.J., Martens, J.H., Rao, N.A., Aghajanirefah, A., et al. (2014). mTOR- and HIF-1alpha-mediated aerobic glycolysis as metabolic basis for trained immunity. Science 345, 1250684.

Connolly, N.M., Dussmann, H., Anilkumar, U., Huber, H.J., and Prehn, J.H. (2014). Single-cell imaging of bioenergetic responses to neuronal excitotoxicity and oxygen and glucose deprivation. J Neurosci 34, 10192-10205.

Dougan, M., and Dranoff, G. (2009). Immune therapy for cancer. Annu Rev Immunol 27, 83-117.

Everts, B., Amiel, E., Huang, S.C., Smith, A.M., Chang, C.H., Lam, W.Y., Redmann, V., Freitas, T.C., Blagih, J., van der Windt, G.J., et al. (2014). TLR-driven early glycolytic reprogramming via the kinases TBK1-IKKvarepsilon supports the anabolic demands of dendritic cell activation. Nat Immunol 15, 323-332.

Everts, B., Amiel, E., van der Windt, G.J., Freitas, T.C., Chott, R., Yarasheski, K.E., Pearce, E.L., and Pearce, E.J. (2012). Commitment to glycolysis sustains survival of NO-producing inflammatory dendritic cells. Blood 120, 1422-1431.

Ganeshan, K., and Chawla, A. (2014). Metabolic regulation of immune responses. Annu Rev Immunol 32, 609-634.

Guilliams, M., and Malissen, B. (2015). A Death Notice for In-Vitro-Generated GM-CSF Dendritic Cells? Immunity 42, 988-990.

Helft, J., Bottcher, J., Chakravarty, P., Zelenay, S., Huotari, J., Schraml, B.U., Goubau, D., and Reis e Sousa, C. (2015). GM-CSF Mouse Bone Marrow Cultures Comprise a Heterogeneous Population of CD11c(+)MHCII(+) Macrophages and Dendritic Cells. Immunity 42, 1197-1211.

Helft, J., Bottcher, J.P., Chakravarty, P., Zelenay, S., Huotari, J., Schraml, B.U., Goubau, D., and Reis, E.S.C. (2016). Alive but Confused: Heterogeneity of CD11c(+) MHC Class II(+) Cells in GM-CSF Mouse Bone Marrow Cultures. Immunity 44, 3-4.

Jewett, M.C., Miller, M.L., Chen, Y., and Swartz, J.R. (2009). Continued protein synthesis at low [ATP] and [GTP] enables cell adaptation during energy limitation. J Bacteriol 191, 1083-1091.

Kelly, B., and O'Neill, L.A. (2015). Metabolic reprogramming in macrophages and dendritic cells in innate immunity. Cell Res 25, 771-784.

Lindqvist, L.M., Tandoc, K., Topisirovic, I., and Furic, L. (2017). Cross-talk between protein synthesis, energy metabolism and autophagy in cancer. Curr Opin Genet Dev 48, 104-111.

Liu, H., Kurtoglu, M., Leon-Annicchiarico, C.L., Munoz-Pinedo, C., Barredo, J., Leclerc, G., Merchan, J., Liu, X., and Lampidis, T.J. (2016). Combining 2-deoxy-D-glucose with fenofibrate leads to tumor cell death mediated by simultaneous induction of energy and ER stress. Oncotarget 7, 36461-36473.

Lutz, M.B., Inaba, K., Schuler, G., and Romani, N. (2016). Still Alive and Kicking: In-Vitro-Generated GM-CSF Dendritic Cells! Immunity 44, 1-2.

Lutz, M.B., Strobl, H., Schuler, G., and Romani, N. (2017). GM-CSF Monocyte-Derived Cells and Langerhans Cells As Part of the Dendritic Cell Family. Front Immunol 8, 1388.

MacIver, N.J., Michalek, R.D., and Rathmell, J.C. (2013). Metabolic regulation of T lymphocytes. Annu Rev Immunol 31, 259-283.

Marquez, S., Fernandez, J.J., Teran-Cabanillas, E., Herrero, C., Alonso, S., Azogil, A., Montero, O., Iwawaki, T., Cubillos-Ruiz, J.R., Fernandez, N., et al. (2017). Endoplasmic Reticulum Stress Sensor IRE1alpha Enhances IL-23 Expression by Human Dendritic Cells. Front Immunol 8, 639.

Merad, M., Sathe, P., Helft, J., Miller, J., and Mortha, A. (2013). The dendritic cell lineage: ontogeny and function of dendritic cells and their subsets in the steady state and the inflamed setting. Annu Rev Immunol 31, 563-604.

Miller, A., Nagy, C., Knapp, B., Laengle, J., Ponweiser, E., Groeger, M., Starkl, P., Bergmann, M., Wagner, O., and Haschemi, A. (2017). Exploring Metabolic Configurations of Single Cells within Complex Tissue Microenvironments. Cell Metab 26, 788-800 e786.

Neijssel, O.M., M. J. Teixeira de Mattos, and D. W. Tempest (1996). Growth yield and energy distribution.

Newick, K., O'Brien, S., Moon, E., and Albelda, S.M. (2017). CAR T Cell Therapy for Solid Tumors. Annu Rev Med 68, 139-152.

Oren, R., Farnham, A.E., Saito, K., Milofsky, E., and Karnovsky, M.L. (1963). Metabolic patterns in three types of phagocytizing cells. J Cell Biol 17, 487-501.

Page, D.B., Postow, M.A., Callahan, M.K., Allison, J.P., and Wolchok, J.D. (2014). Immune modulation in cancer with antibodies. Annu Rev Med 65, 185-202.

Schimmel, P. (1993). GTP hydrolysis in protein synthesis: two for Tu? Science 259, 1264-1265.

Schmidt, E.K., Clavarino, G., Ceppi, M., and Pierre, P. (2009). SUnSET, a nonradioactive method to monitor protein synthesis. Nat Methods 6, 275-277.

Steinman, R.M. (2012). Decisions about dendritic cells: past, present, and future. Annu Rev Immunol 30, 1-22.

Steinman, R.M., Hawiger, D., and Nussenzweig, M.C. (2003). Tolerogenic dendritic cells. Annu Rev Immunol 21, 685-711.

Suganya, R., Chakraborty, A., Miriyala, S., Hazra, T.K., and Izumi, T. (2015). Suppression of oxidative phosphorylation in mouse embryonic fibroblast cells deficient in apurinic/apyrimidinic endonuclease. DNA Repair (Amst) 27, 40-48.

Villani, A.C., Satija, R., Reynolds, G., Sarkizova, S., Shekhar, K., Fletcher, J., Griesbeck, M., Butler, A., Zheng, S., Lazo, S., et al. (2017). Single-cell RNA-seq reveals new types of human blood dendritic cells, monocytes, and progenitors. Science 356.

Wallace, D.C., Fan, W., and Procaccio, V. (2010). Mitochondrial energetics and therapeutics. Annu Rev Pathol 5, 297-348.

Wolf, A.J., Reyes, C.N., Liang, W., Becker, C., Shimada, K., Wheeler, M.L., Cho, H.C., Popescu, N.I., Coggeshall, K.M., Arditi, M., et al. (2016). Hexokinase Is an Innate Immune Receptor for the Detection of Bacterial Peptidoglycan. Cell 166, 624-636.

Zhang, J., Nuebel, E., Wisidagama, D.R., Setoguchi, K., Hong, J.S., Van Horn, C.M., Imam, S.S., Vergnes, L., Malone, C.S., Koehler, C.M., et al. (2012). Measuring energy metabolism in cultured cells, including human pluripotent stem cells and differentiated cells. Nat Protoc 7, 1068-1085.

Zhang, L., Su, J., Xie, Q., Zeng, L., Wang, Y., Yi, D., Yu, Y., Liu, S., Li, S., and Xu, Y. (2015). 2-Deoxy-d-Glucose Sensitizes Human Ovarian Cancer Cells to Cisplatin by Increasing ER Stress and Decreasing ATP Stores in Acidic Vesicles. J Biochem Mol Toxicol 29, 572-578.

Zhang, P., McGrath, B., Li, S., Frank, A., Zambito, F., Reinert, J., Gannon, M., Ma, K., McNaughton, K., and Cavener, D.R. (2002). The PERK eukaryotic initiation factor 2 alpha kinase is required for the development of the skeletal system, postnatal growth, and the function and viability of the pancreas. Mol Cell Biol 22, 3864-3874.

## Claims

1. An in vitro method of profiling the energetic metabolism profile of a population of cells comprising:

   i) providing four samples [S1], [S2], [S3] and [S4] of said population of cells
   ii) measuring the protein synthesis level [LCo] in sample [S1] in absence of any inhibitor;
   iii) contacting the sample [S2] with an inhibitor [A] of the production of the energy resulting from glycolysis and the oxidative phosphorylation of glucose-derived pyruvate and measuring the protein synthesis level [LA] in said sample;

iv) contacting the sample [S3] with an inhibitor [B] of the production of the energy resulting from TCA cycle and oxidative phosphorylation comprising pyruvate oxidation, oxidation of fatty acids and oxidation of amino acids and measuring the protein synthesis level [LB] in said sample;

v) contacting the sample [S4] cells with both inhibitors [A] and [B] and measuring the protein synthesis level [L(A+B)] in said sample

vi) assessing the glucose dependency of the population of cells; wherein the glucose dependency of the cells is assessed by calculating the formula (I):

$$\text{Glucose dependency} = ([LCo]-[LA]) \ / \ ([LCo]-[L(A+B)]) \ x \ 100 \ (I)$$

vii) assessing the mitochondrial dependency of the population of cells; wherein the mitochondrial dependency of the cells is assessed by calculating the formula (II):

$$\text{Mitochondrial dependency} = ([LCo]-[LB]) \ / \ ([LCo]-[L(A+B)]) \ x \ 100 \ (II)$$

viii) assessing the glycolytic capacity of the population of cells; wherein the glycolytic capacity of the cells is assessed by calculating the formula (III):

$$\text{Glycolytic capacity} = (1 \ - \ ([LCo]-[LB]) \ / \ ([LCo]-[L(A+B)])) \ x \ 100 \ (III)$$

ix) assessing the capacity for oxidation of fatty acids and oxidation of amino acids of the population of cells, wherein the oxidation of fatty acids and oxidation of amino acids capacity is assessed by calculating the formula (IV):

Oxidation of fatty acids & oxidation of amino acids capacity = $(1 - ([LCo]-[LA]) \ / \ ([LCo]-[L(A+B)])) \times 100$    (IV)

and

x) finally determining the energetic metabolism profile of the population of cells.

2. The method of claim 1 wherein the population of cells consists of a homogeneous population of cells or a heterogeneous population of cells or results from any biological sample obtained from a subject

3. The method of claim 1 wherein the population of cells comprises immune cells, such as lymphocytes (such as B cells and T cells); natural killer cells; myeloid cells (such as monocytes, macrophages and dendritic cells), neutrophils, eosinophils, mast cells, basophils, or granulocytes.

4. The method of claim 1 wherein the inhibitor [A] is selected from the group consisting of 2-Deoxy-Glucose, 2-[N-(7-Nitrobenz-2-oxa-1,3-diaxol-4-yl)amino]-2-deoxyglucose/2-NBDG, Phloretin, 3-Bromophyruvic acid, Iodoacetate, Fluoride and 6-Aminonicotinamide.

5. The method of claim 1 wherein step iii) is performed in presence of an amount of pyruvate or acetate.

6. The method of claim 1 wherein the inhibitor [B] is selected from the group consisting of Oligomycin (A/B/C/D/E//F and derivates), Rotenone, Carbonyl cyanide-p-trifluoromethoxyphenylhydrazone/FCCP, Trimetazidine/TMZ, 2[6(4-chlorophenoxy)hexyl]oxirane-2-carboxylate/Etomoxir, Bis-2-(5-phenylacetamido-1,3,4-thiadiazol-2-yl)ethyl sulfide/BPTES, inhibitors of wild type or mutant enzymes of mitochondrial metabolism pathways, and enasidenib.

7. The method of claim 1 which further comprises the steps of:

- providing a further sample [S5] of the population of cells
- contacting said sample with an inhibitor [C] of the production of the energy resulting from oxidation of fatty acids and measuring the protein synthesis level [LC] in said sample and,
- assessing the dependency of oxidation of fatty acids of the population cells, wherein the dependency of oxidation of fatty acids is assessed by calculating the formula (V):

$$\text{Dependency of oxidation of fatty acids} = ([LCo]-[LC]) / ([LCo]-[L(A+B)]) \times 100 \qquad (V)$$

8. The method of claim 7 wherein, the inhibitor [C] is selected from the group consisting of Trimetazidine/TMZ and 2[6(4-chlorophenoxy)hexyl]oxirane-2-carboxylate/Etomoxir.

9. The method of claim 1 which further comprises the steps of

   - providing a further sample [S6] of the population of cells
   - contacting the sample with an inhibitor [D] of the production of the energy resulting from oxidation of amino acids and measuring the protein synthesis level [LD] in the sample and
   - assessing the dependency of oxidation of amino acids of the population cells, wherein the dependency of oxidation of amino acids is assessed by calculating the formula (VI):

$$\text{Dependency of oxidation of amino acids} = ([LCo] - [LD]) / ([LCo]-[L(A+B)]) \times 100 \qquad (VI)$$

10. The method of claim 9, wherein the inhibitor [D] is selected from the group consisting of Bis-2-(5-phenylacetamido-1,3,4-thiadiazol-2-yl)ethyl sulfide/BPTES, Aminooxyacetic acid/AOA and epigallocatechin-3-gallate/EGCG.

11. The method of claim 1 wherein the protein synthesis level sample is determined by contacting the sample with an amount of puromycin and then after with an amount of monoclonal antibodies specific for puromycin that are typically conjugated with a detectable label.

12. The method of claim 11 wherein the label is a heavy metal, a fluorescent label, a chemiluminescent label, an enzyme label, a bioluminescent label, a colloidal gold, or a DNA-barcode oligonucleotide.

13. The method of claim 11 wherein the protein synthesis level is assessed by cytometry, CyTOF or Cite-seq.

14. The method of claim 1 which further comprises identifying a particular cell type in said population of cells, in particular by using of a panel of binding partners specific for some cell surface markers of interest (e.g. BCR, CD19 or CD20 for B cells and TCR, CD4, CD8, CD25 for T cells).

15. The method of claim 1 wherein determining the energetic metabolism profile of the cell according step x) comprises concluding that said cell presents a respiratory profile or a glycolysis profile

16. Use of the method of claim 1 for diagnostic purposes.

17. Use of the method of claim 1 for predicting the survival time of a patient suffering from cancer.

18. Use of the method of claim 1 for predicting whether a subject suffering from cancer will be eligible to a therapy, in particular chemotherapy or immunotherapy.

19. A kit for performing the method of claim 1 comprising:

   - an amount of inhibitor [A] of the production of the energy resulting from glycolysis and the oxidative phosphorylation of glucose-derived pyruvate,
   - an amount of inhibitor [B] of the production of the energy resulting from TCA cycle and oxidative phosphorylation comprising pyruvate oxidation, oxidation of fatty acids and oxidation of amino acids,
   - an amount of puromycin,
   - an amount of monoclonal antibodies specific to puromycin,
   - optionally an amount of inhibitor [C] of the production of the energy resulting from oxidation of fatty acids,
   - optionally an amount of inhibitor [D] of the production of the energy resulting from oxidation of amino acids,
   - optionally an amount of pyruvate,
   - optionally an amount of acetate,
   - optionally a panel of antibodies for cell sorting, and
   - optionally a software package for calculating the different formulas (I-VI) suitable for assessing the metabolic profile (i.e. different dependencies).

**Patentansprüche**

1. In-vitro-Verfahren zur Profilierung des energetischen Stoffwechselprofils einer Zellpopulation, umfassend:

   i) Bereitstellen von vier Proben [S1], [S2], [S3] und [S4] der Zellpopulation
   ii) Messen des Proteinsyntheseniveaus [LCo] in Probe [S1] in Abwesenheit jeglichen Inhibitors;
   iii) Inkontaktbringen der Probe [S2] mit einem Inhibitor [A] der Energieproduktion, die aus Glykolyse und der oxidativen Phosphorylierung von Glucoseabgeleiteten Pyruvat resultiert, und Messen des Proteinsyntheseniveaus [LA] in der Probe;
   iv) Inkontaktbringen der Probe [S3] mit einem Inhibitor [B] der Energieproduktion, die aus TCA-Zyklus und oxidativer Phosphorylierung resultiert, die Pyruvatoxidation, Oxidation von Fettsäuren und Oxidation von Aminosäuren umfasst, und Messen des Proteinsyntheseniveaus [LB] in der Probe;
   v) Inkontaktbringen der Proben- [S4] Zellen mit beiden Inhibitoren [A] und [B] und Messen des Proteinsyntheseniveaus [L(A+B)] in der Probe;
   vi) Ermitteln der Glucoseabhängigkeit der Zellpopulation; wobei die Glucoseabhängigkeit der Zellen durch Berechnen der Formel (I) ermittelt wird:

   $$\text{Glucoseabhängigkeit} = ([LCo]-[LA]) / ([LCo]-[L(A+B)]) \times 100 \ (I)$$

   vii) Ermitteln der mitochondrialen Abhängigkeit der Zellpopulation; wobei die mitochondriale Abhängigkeit der Zellen durch Berechnen der Formel (II) ermittelt wird:

   $$\text{Mitochondriale Abhängigkeit} = ([LCo]-[LB]) / ([LCo]-[L(A+B)]) \times 100 \ (II)$$

   viii) Ermitteln der glykolytischen Kapazität der Zellpopulation; wobei die glykolytische Kapazität der Zellen durch Berechnen der Formel (III) ermittelt wird:

   $$\text{Glykolytische Kapazität} = (1 - ([LCo]-[LB]) / ([LCo]-[L(A+B)])) \times 100 \ (III)$$

   ix) Ermitteln der Kapazität der Zellpopulation zur Oxidation von Fettsäuren und zur Oxidation von Aminosäuren, wobei die Kapazität zur Oxidation von Fettsäuren und zur Oxidation von Aminosäuren durch Berechnen der Formel (IV) ermittelt wird:

   $$\text{Kapazität zur Oxidation von Fettsäuren \& Oxidation von Aminosäuren} = (1- ([LCo]-[LA]) / ([LCo]-[L(A+B)])) \times 100$$

   und
   x) schließlich Bestimmen des energetischen Stoffwechselprofils der Zellpopulation.

2. Verfahren nach Anspruch 1, wobei die Zellpopulation aus einer homogenen Zellpopulation oder einer heterogenen Zellpopulation besteht oder aus einer biologischen Probe resultiert, die von einem Subjekt erhalten wurde.

3. Verfahren nach Anspruch 1, wobei die Zellpopulation Immunzellen wie Lymphozyten (wie B-Zellen und T-Zellen), natürliche Killerzellen, myeloide Zellen (wie Monozyten, Makrophagen und dendritische Zellen), Neutrophile, Eosinophile, Mastzellen, Basophile oder Granulozyten umfasst.

4. Verfahren nach Anspruch 1, wobei der Inhibitor [A] ausgewählt ist aus der Gruppe bestehend aus 2-Deoxy-Glucose, 2-[N-(7-Nitrobenz-2-oxa-1,3-diaxol-4-yl)amino]-2-deoxyglucose/2-NBDG, Phloretin, 3-Bromphyruvatsäure, Iodacetat, Fluorid und 6-Aminonicotinamid.

5. Verfahren nach Anspruch 1, wobei Schritt iii) in Gegenwart einer Menge Pyruvat oder Acetat durchgeführt wird.

6. Verfahren nach Anspruch 1, wobei der Inhibitor [B] ausgewählt ist aus der Gruppe bestehend aus Oligomycin (A/B/C/D/E//F und Derivate), Rotenon, Carbonylcyanid-p-trifluormethoxyphenylhydrazon/FCCP, Trimetazidin/TMZ, 2[6(4-Chlorphenoxy)hexyl]oxiran-2-carboxylat/Etomoxir, Bis-2-(5-phenylacetamido-1,3,4-thiadiazol-2-yl)ethylsulfid/BPTES, Inhibitoren von Wildtyp- oder mutierten Enzymen mitochondrialer Stoffwechselwege und Enasidenib.

7. Verfahren nach Anspruch 1,das weiter die folgenden Schritte umfasst:

- Bereitstellen einer weiteren Probe [S5] der Zellpopulation
- Inkontaktbringen der Probe mit einem Inhibitor [C] der Energieproduktion, die aus Oxidation von Fettsäuren resultiert, und Messen des Proteinsyntheseniveaus [LC] in der Probe und
- Ermitteln der Abhängigkeit einer Oxidation von Fettsäuren von den Populationszellen, wobei die Abhängigkeit einer Oxidation von Fettsäuren durch Berechnen der Formel (V) ermittelt wird:

$$\text{Abhängigkeit einer Oxidation von Fettsäuren} = ([LCo]-[LC]) / ([LCo]-[L(A+B)]) \times 100 \qquad (V)$$

8. Verfahren nach Anspruch 7, wobei der Inhibitor [C] ausgewählt ist aus der Gruppe bestehend aus Trimetazidin/TMZ und 2[6(4-Chlorphenoxy)hexyl]oxiran-2-carboxylat/Etomoxir.

9. Verfahren nach Anspruch 1, das weiter die Schritte umfasst von

- Bereitstellen einer weiteren Probe [S6] der Zellpopulation
- Inkontaktbringen der Probe mit einem Inhibitor [D] der Energieproduktion, die aus Oxidation von Aminosäuren resultiert, und Messen des Proteinsyntheseniveaus [LD] in der Probe und
- Ermitteln der Abhängigkeit einer Oxidation von Aminosäuren von den Populationszellen, wobei die Abhängigkeit einer Oxidation von Aminosäuren durch Berechnen der Formel (VI) ermittelt wird:

$$\text{Abhängigkeit einer Oxidation von Aminosäuren} = ([LCo]-[LD]) / ([LCo]-[L(A+B)]) \times 100 \qquad (VI)$$

10. Verfahren nach Anspruch 9, wobei der Inhibitor [D] ausgewählt ist aus der Gruppe bestehend aus Bis-2-(5-phenylacetamido-1,3,4-thiadiazol-2-yl)ethylsulfid/BPTES, Aminooxyessigsäure/AO A und Epigallocatechin-3-gallat/EGCG.

11. Verfahren nach Anspruch 1, wobei das Proteinsyntheseniveau der Probe bestimmt wird, indem die Probe mit einer Menge Puromycin und dann mit einer Menge für Puromycin spezifischer monoklonaler Antikörper in Kontakt gebracht wird, die typischerweise mit einer nachweisbaren Markierung konjugiert sind.

12. Verfahren nach Anspruch 11, wobei die Markierung ein Schwermetall, eine Fluoreszenzmarkierung, eine Chemilumineszenzmarkierung, eine Enzymmarkierung, eine Biolumineszenzmarkierung, ein kolloidales Gold oder ein DNA-Barcode-Oligonukleotid ist.

13. Verfahren nach Anspruch 11, wobei das Proteinsyntheseniveau mittels Zytometrie, CyTOF oder Cite-seq ermittelt wird.

14. Verfahren nach Anspruch 1, das weiter Identifizieren eines bestimmten Zelltyps in der Zellpopulation umfasst, insbesondere durch Verwendung einer Reihe von Bindungspartnern, die für bestimmte Zelloberflächenmarker von Interesse (z. B. BCR, CD19 oder CD20 für B-Zellen und TCR, CD4, CD8, CD25 für T-Zellen) spezifisch sind.

15. Verfahren nach Anspruch 1, wobei Bestimmen des energetischen Stoffwechselprofils der Zelle gemäß Schritt x) Schlussfolgern umfasst, dass die Zelle ein Atmungsprofil oder ein Glykolyseprofil aufweist

16. Verwendung des Verfahrens nach Anspruch 1 für diagnostische Zwecke.

17. Verwendung des Verfahrens nach Anspruch 1 zum Vorhersagen der Überlebenszeit eines an Krebs erkrankten Patienten.

18. Verwendung des Verfahrens nach Anspruch 1 zum Vorhersagen, ob ein an Krebs erkranktes Subjekt für eine Therapie, insbesondere Chemotherapie oder Immuntherapie, in Frage kommt.

**19.** Kit zum Durchführen des Verfahrens nach Anspruch 1, umfassend:

- eine Menge an Inhibitor [A] der Energieproduktion, die aus Glykolyse und der oxidativen Phosphorylierung von Glucose-abgeleitetem Pyruvat resultiert,
- eine Menge an Inhibitor [B] der Energieproduktion, die aus TCA-Zyklus und oxidativer Phosphorylierung resultiert, die Pyruvatoxidation, Oxidation von Fettsäuren und Oxidation von Aminosäuren umfasst,
- eine Menge Puromycin,
- eine Menge an monoklonalen Antikörpern, die spezifisch gegen Puromycin sind,
- optional eine Menge an Inhibitor [C] der Energieproduktion, die aus Oxidation von Fettsäuren resultiert,
- optional eine Menge an Inhibitor [D] der Energieproduktion, die aus Oxidation von Aminosäuren resultiert,
- optional eine Menge Pyruvat,
- optional eine Menge Acetat,
- optional eine Reihe von Antikörpern zur Zellsortierung und
- optional ein Softwarepaket zum Berechnen der verschiedenen Formeln (I-VI), die zum Ermitteln des Stoffwechselprofils (d. h. verschiedener Abhängigkeiten) geeignet sind.

**Revendications**

**1.** Procédé in vitro de profilage du profil de métabolisme énergétique d'une population de cellules consistant à :

i) fournir quatre échantillons [S1], [S2], [S3] et [S4] de ladite population de cellules
ii) mesurer le niveau de synthèse de protéine [LCo] dans l'échantillon [S1] en l'absence de tout inhibiteur ;
iii) mettre en contact l'échantillon [S2] avec un inhibiteur [A] de la production de l'énergie résultant de la glycolyse et de la phosphorylation oxydative du pyruvate dérivé du glucose et de la mesure du niveau de synthèse de protéine [LA] dans ledit échantillon ;
iv) mettre en contact l'échantillon [S3] avec un inhibiteur [B] de la production de l'énergie résultant du cycle TCA et de la phosphorylation oxydative comprenant l'oxydation de pyruvate, l'oxydation d'acides gras et l'oxydation d'acides aminés et mesurer le niveau de synthèse de protéine [LB] dans ledit échantillon ;
v) mettre en contact les cellules d'échantillon [S4] avec les deux inhibiteurs [A] et [B] et mesurer le niveau de synthèse de protéine [L(A+B)] dans ledit échantillon
vi) évaluer la dépendance au glucose de la population de cellules ; dans lequel la dépendance au glucose des cellules est évaluée en calculant la formule (I) :

$$\text{Dépendance au glucose} = ([LCo]-[LA]) / ([LCo]-[L(A+B)]) \times 100 \ (I)$$

vii) évaluer la dépendance mitochondriale de la population de cellules ; dans lequel la dépendance mitochondriale des cellules est évaluée en calculant la formule (II) :

$$\text{Dépendance mitochondriale} = ([LCo]-[LB]) / ([LCo]-[L(A+B)]) \times 100 \ (II)$$

viii) évaluer la capacité glycolytique de la population de cellules ; dans lequel la capacité glycolytique des cellules est évaluée en calculant la formule (III) :

$$\text{Capacité glycolytique} = (1 - ([LCo]-[LB]) / ([LCo]-[L(A+B)])) \times 100 \ (III)$$

ix) évaluer la capacité d'oxydation d'acides gras et d'oxydation d'acides aminés de la population de cellules, dans lequel la capacité d'oxydation d'acides gras et d'oxydation d'acides aminés est évaluée en calculant la formule (IV) :

$$\text{Capacité d'oxydation d'acides gras et d'oxydation d'acides aminés} = (1- ([LCo]-[LA]) / ([LCo]-[L(A+B)])) \times 100 \quad (IV)$$

et
x) déterminer enfin le profil de métabolisme énergétique de la population de cellules.

**2.** Procédé selon la revendication 1, dans lequel la population de cellules consiste en une population homogène de cellules ou une population hétérogène de cellules ou résulte de tout échantillon biologique obtenu à partir d'un sujet.

3. Procédé selon la revendication 1, dans lequel la population de cellules comprend des cellules immunitaires, telles que des lymphocytes (tels que des cellules B et des cellules T) ; cellules tueuses naturelles ; cellules myéloïdes (telles que des monocytes, macrophages et cellules dendritiques), neutrophiles, éosinophiles, mastocytes, basophiles ou granulocytes.

4. Procédé selon la revendication 1, dans lequel l'inhibiteur [A] est sélectionné à partir du groupe consistant en 2-désoxy-glucose, 2-[N-(7-nitrobenz-2-oxa-1,3-diaxol-4-yl)amino]-2-désoxyglucose/2-NBDG, phlorétine, acide 3-bromophyruvique, iodoacétate, fluorure et 6-aminonicotinamide.

5. Procédé selon la revendication 1, dans lequel l'étape iii) est mise en œuvre en présence d'une quantité de pyruvate ou d'acétate.

6. Procédé selon la revendication 1, dans lequel l'inhibiteur [B] est sélectionné à partir du groupe consistant en oligomycine (A/B/C/D/E//F et dérivés), roténone, carbonyle cyanide-p-trifluoromethoxyphenylhydrazone/FCCP, trimétazidine/TMZ, 2[6(4-chlorophénoxy)hexyl]oxirane-2-carboxylate/etomoxir, bis-2-(5-phénylacétamido-1,3,4-thiadiazol-2-yl)éthyle sulfide/BPTES, inhibiteurs d'enzymes sauvages ou mutantes des voies de métabolisme mitochondrial et enasidenib.

7. Procédé selon la revendication 1, qui comprend en outre les étapes consistant à :

- fournir un échantillon supplémentaire [S5] de la population de cellules
- mettre en contact ledit échantillon avec un inhibiteur [C] de la production de l'énergie résultant de l'oxydation d'acides gras et mesurer le niveau de synthèse de protéine [LC] dans ledit échantillon et,
- évaluer la dépendance de l'oxydation d'acides gras des cellules de population, dans lequel la dépendance de l'oxydation d'acides gras est évaluée en calculant la formule (V) :

$$\text{Dépendance de l'oxydation d'acides gras} = ([LCo]-[LC]) / ([LCo]-[L(A+B)]) \times 100 \quad (V)$$

8. Procédé selon la revendication 7, dans lequel l'inhibiteur [C] est sélectionné à partir du groupe consistant en trimétazidine/TMZ et 2[6(4-chlorophénoxy)hexyl]oxirane-2-carboxylate/etomoxir.

9. Procédé selon la revendication 1, qui comprend en outre les étapes consistant à

- fournir un échantillon supplémentaire [S6] de la population de cellules
- mettre en contact l'échantillon avec un inhibiteur [D] de la production de l'énergie résultant de l'oxydation d'acides aminés et mesurer le niveau de synthèse de protéine [LD] dans l'échantillon et
- évaluer la dépendance de l'oxydation d'acides aminés des cellules de population, dans lequel la dépendance de l'oxydation d'acides aminés est évaluée en calculant la formule (VI) :

$$\text{Dépendance de l'oxydation d'acides aminés} = ([LCo]-[LD]) / ([LCo]-[L(A+B)]) \times 100 \quad (VI)$$

10. Procédé selon la revendication 9, dans lequel l'inhibiteur [D] est sélectionné à partir du groupe consistant en bis-2-(5-phénylacétamido-1,3,4-thiadiazol-2-yl)éthyl sulfide/BPTES, acide aminooxyacétique/AO A et épigallocatéchine-3-gallate/EGCG

11. Procédé selon la revendication 1, dans lequel le niveau de synthèse de protéine de l'échantillon est déterminé en mettant en contact l'échantillon avec une quantité de puromycine, puis avec une quantité d'anticorps monoclonaux spécifiques de la puromycine qui sont typiquement conjugués à un marqueur détectable.

12. Procédé selon la revendication 11, dans lequel le marqueur est un métal lourd, un marqueur fluorescent, un marqueur chimioluminescent, un marqueur enzymatique, un marqueur bioluminescent, un or colloïdal ou un oligonucléotide de code-barres d'ADN.

13. Procédé selon la revendication 11, dans lequel le niveau de synthèse de protéine est évalué par cytométrie, CyTOF ou Cite-seq.

14. Procédé selon la revendication 1, qui comprend en outre les étapes consistant à identifier un type de cellule particulier dans ladite population de cellules, en particulier en utilisant un panel de partenaires de liaison spécifiques de certains marqueurs de surface de cellule d'intérêt (par ex. BCR, CD19 ou CD20 pour les cellules B et TCR, CD4, CD8, CD25 pour les cellules T).

15. Procédé selon la revendication 1, dans lequel la détermination du profil de métabolisme énergétique de la cellule selon l'étape x) consiste à conclure que ladite cellule présente un profil respiratoire ou un profil de glycolyse.

16. Utilisation du procédé selon la revendication 1 à des fins de diagnostic.

17. Utilisation du procédé selon la revendication 1 pour prédire la durée de survie d'un patient souffrant d'un cancer.

18. Utilisation du procédé selon la revendication 1 pour prédire si un sujet atteint d'un cancer sera éligible à une thérapie, notamment une chimiothérapie ou une immunothérapie.

19. Kit pour mettre en œuvre le procédé selon la revendication 1, comprenant :

- une quantité d'inhibiteur [A] de la production de l'énergie résultant de la glycolyse et de la phosphorylation oxydative de pyruvate dérivé du glucose,
- une quantité d'inhibiteur [B] de la production de l'énergie résultant du cycle TCA et de la phosphorylation oxydative comprenant l'oxydation de pyruvate, l'oxydation d'acides gras et l'oxydation d'acides aminés,
- une quantité de puromycine,
- une quantité d'anticorps monoclonaux spécifiques de la puromycine,
- facultativement une quantité d'inhibiteur [C] de la production de l'énergie résultant de l'oxydation d'acides gras,
- facultativement une quantité d'inhibiteur [D] de la production de l'énergie résultant de l'oxydation d'acides aminés,
- facultativement une quantité de pyruvate,
- facultativement une quantité d'acétate,
- facultativement un panel d'anticorps pour le tri cellulaire, et
- facultativement un progiciel pour calculer les différentes formules (I-VI) adaptées à l'évaluation du profil métabolique (c'est-à-dire différentes dépendances).

Figure 1

Figure 2

Figures 3G-J

**A**

WT or. CD11c-*CRE PERK*[flox/flox]

↓

Bone marrow

↓

+FLT3L
(7 days)

↓

ZeNITH

↓

Glucose dependence:
WT vs K.O

**Figure 4A**

**Figures 4B & 4C**

Figure 5A

**Figure 5B**

Figure 5C

**Figure 5D**

**Figure 6A, 6B and 6C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5866366 A, Nazarenko
- US 5800996 A, Lee
- US 5696157 A
- US 6130101 A
- US 6716979 B
- US 4774339 A
- US 5187288 A
- US 5248782 A
- US 5274113 A
- US 5338854 A
- US 5451663 A
- US 5433896 A
- US 5132432 A
- US 5830912 A
- FR 0856499

### Non-patent literature cited in the description

- **SCHMIDT, E.K.** ; **CLAVARINO, G.** ; **CEPPI, M.** ; **PIERRE, P.** SUnSET, a nonradioactive method to monitor protein synthesis.. *Nat Methods*, 2009, vol. 6, 275-277
- **HEYDUK** ; **HEYDUK**. *Analyt. Biochem.*, 1997, vol. 248, 216-27
- *J. Biol. Chem.*, 1999, vol. 274, 3315-22
- **KENNEDY et al.** *Clin. Chim. Acta*, 1976, vol. 70, 1
- **SCHURS et al.** *Clin. Chim. Acta*, 1977, vol. 81, 1
- **SHIH et al.** *Int'l J. Cancer*, 1990, vol. 46, 1101
- **STEIN et al.** *Cancer Res.*, 1990, vol. 50, 1330
- **CHEUNG et al.** Screening: CyTOF-the next generation of cell detection. *Nature Reviews Rheumatology*, 2011, vol. 7, 502-503
- **BENDALL et al.** Single-Cell Mass Cytometry of Differential Immune and Drug Responses Across a Human Hematopoietic Continuum. *Science*, 2011, vol. 332 (6030), 687-696
- **STOECKIUS, MARLON et al.** Simultaneous epitope and transcriptome measurement in single cells.. *Nature methods*, 2017, vol. 14 (9), 865
- **ASSMANN, N.** ; **FINLAY, D.K.** Metabolic regulation of immune responses: therapeutic opportunities.. *J Clin Invest*, 2016, vol. 126, 2031-2039
- **BUTTGEREIT, F** ; **BRAND, M.D.** A hierarchy of ATP-consuming processes in mammalian cells.. *Biochem J*, 1995, vol. 312 (1), 163-167
- **CHENG, S.C.** ; **QUINTIN, J.** ; **CRAMER, R.A.** ; **SHEPARDSON, K.M.** ; **SAEED, S.** ; **KUMAR, V** ; **GIAMARELLOS-BOURBOULIS, E.J.** ; **MARTENS, J.H.** ; **RAO, N.A.** ; **AGHAJANIREFAH, A. et al.** mTOR- and HIF-1alpha-mediated aerobic glycolysis as metabolic basis for trained immunity.. *Science*, 2014, vol. 345, 1250684
- **CONNOLLY, N.M.** ; **DUSSMANN, H.** ; **ANILKUMAR, U.** ; **HUBER, H.J.** ; **PREHN, J.H.** Single-cell imaging of bioenergetic responses to neuronal excitotoxicity and oxygen and glucose deprivation.. *J Neurosci*, 2014, vol. 34, 10192-10205
- **DOUGAN, M** ; **DRANOFF, G.** Immune therapy for cancer.. *Annu Rev Immunol*, 2009, vol. 27, 83-117
- **EVERTS, B.** ; **AMIEL, E.** ; **HUANG, S.C.** ; **SMITH, A.M.** ; **CHANG, C.H.** ; **LAM, W.Y.** ; **REDMANN, V.** ; **FREITAS, T.C.** ; **BLAGIH, J.** ; **VAN DER WINDT, G.J. et al.** TLR-driven early glycolytic reprogramming via the kinases TBK1-IKKvarepsilon supports the anabolic demands of dendritic cell activation.. *Nat Immunol*, 2014, vol. 15, 323-332
- **EVERTS, B.** ; **AMIEL, E.** ; **VAN DER WINDT, G.J.** ; **FREITAS, T.C.** ; **CHOTT, R.** ; **YARASHESKI, K.E** ; **PEARCE, E.L.** ; **PEARCE, E.J.** Commitment to glycolysis sustains survival of NO-producing inflammatory dendritic cells.. *Blood*, 2012, vol. 120, 1422-1431
- **GANESHAN, K.** ; **CHAWLA, A.** Metabolic regulation of immune responses.. *Annu Rev Immunol*, 2014, vol. 32, 609-634
- **GUILLIAMS, M.** ; **MALISSEN, B.** A Death Notice for In-Vitro-Generated GM-CSF Dendritic Cells?. *Immunity*, 2015, vol. 42, 988-990
- **HELFT, J.** ; **BOTTCHER, J.** ; **CHAKRAVARTY, P.** ; **ZELENAY, S.** ; **HUOTARI, J.** ; **SCHRAML, B.U.** ; **GOUBAU, D.** ; **REIS E SOUSA, C.** GM-CSF Mouse Bone Marrow Cultures Comprise a Heterogeneous Population of CD11c(+)MHCII(+) Macrophages and Dendritic Cells.. *Immunity*, 2015, vol. 42, 1197-1211

- **HELFT, J.** ; **BOTTCHER, J.P.** ; **CHAKRAVARTY, P.** ; **ZELENAY, S.** ; **HUOTARI, J.** ; **SCHRAML, B.U.** ; **GOUBAU, D.** ; **REIS, E.S.C.** Alive but Confused: Heterogeneity of CD11c(+) MHC Class II(+) Cells in GM-CSF Mouse Bone Marrow Cultures.. *Immunity*, 2016, vol. 44, 3-4
- **JEWETT, M.C.** ; **MILLER, M.L.** ; **CHEN, Y.** ; **SWARTZ, J.R.** Continued protein synthesis at low [ATP] and [GTP] enables cell adaptation during energy limitation.. *J Bacteriol*, 2009, vol. 191, 1083-1091
- **KELLY, B.** ; **O'NEILL, L.A.** Metabolic reprogramming in macrophages and dendritic cells in innate immunity.. *Cell Res*, 2015, vol. 25, 771-784
- **LINDQVIST, L.M.** ; **TANDOC, K.** ; **TOPISIROVIC, I.** ; **FURIC, L.** Cross-talk between protein synthesis, energy metabolism and autophagy in cancer.. *Curr Opin Genet Dev*, 2017, vol. 48, 104-111
- **LIU, H.** ; **KURTOGLU, M.** ; **LEON-ANNICCHIARICO, C.L.** ; **MUNOZ-PINEDO, C.** ; **BARREDO, J.** ; **LECLERC, G.** ; **MERCHAN, J.** ; **LIU, X.** ; **LAMPIDIS, T.J.** Combining 2-deoxy-D-glucose with fenofibrate leads to tumor cell death mediated by simultaneous induction of energy and ER stress.. *Oncotarget*, 2016, vol. 7, 36461-36473
- **LUTZ, M.B.** ; **INABA, K.** ; **SCHULER, G.** ; **ROMANI, N.** Still Alive and Kicking: In-Vitro-Generated GM-CSF Dendritic Cells!. *Immunity*, 2016, vol. 44, 1-2
- **LUTZ, M.B.** ; **STROBL, H.** ; **SCHULER, G.** ; **ROMANI, N**. GM-CSF Monocyte-Derived Cells and Langerhans Cells As Part of the Dendritic Cell Family.. *Front Immunol*, 2017, vol. 8, 1388
- **MACIVER, N.J.** ; **MICHALEK, R.D** ; **RATHMELL, J.C.** Metabolic regulation of T lymphocytes.. *Annu Rev Immunol*, 2013, vol. 31, 259-283
- **MARQUEZ, S.** ; **FERNANDEZ, J.J.** ; **TERAN-CABANILLAS, E.** ; **HERRERO, C.** ; **ALONSO, S.** ; **AZOGIL, A.** ; **MONTERO, O.** ; **IWAWAKI, T.** ; **CUBILLOS-RUIZ, J.R.** ; **FERNANDEZ, N. et al.** Endoplasmic Reticulum Stress Sensor IRE1alpha Enhances IL-23 Expression by Human Dendritic Cells.. *Front Immunol*, 2017, vol. 8, 639
- **MERAD, M.** ; **SATHE, P.** ; **HELFT, J.** ; **MILLER, J.** ; **MORTHA, A.** The dendritic cell lineage: ontogeny and function of dendritic cells and their subsets in the steady state and the inflamed setting.. *Annu Rev Immunol*, 2013, vol. 31, 563-604
- **MILLER, A.** ; **NAGY, C.** ; **KNAPP, B.** ; **LAENGLE, J.** ; **PONWEISER, E.** ; **GROEGER, M.** ; **STARKL, P.** ; **BERGMANN, M.** ; **WAGNER, O.** ; **HASCHEMI, A.** Exploring Metabolic Configurations of Single Cells within Complex Tissue Microenvironments.. *Cell Metab*, 2017, vol. 26, 788-800, e786
- **NEIJSSEL, O.M.** ; **M. J. TEIXEIRA DE MATTOS** ; **D. W. TEMPEST**. *Growth yield and energy distribution*, 1996
- **NEWICK, K.** ; **O'BRIEN, S.** ; **MOON, E.** ; **ALBELDA, S.M.** CAR T Cell Therapy for Solid Tumors.. *Annu Rev Med*, 2017, vol. 68, 139-152
- **OREN, R.** ; **FARNHAM, A.E.** ; **SAITO, K.** ; **MILOFSKY, E.** ; **KARNOVSKY, M.L**. Metabolic patterns in three types of phagocyting cells.. *J Cell Biol*, 1963, vol. 17, 487-501
- **PAGE, D.B.** ; **POSTOW, M.A.** ; **CALLAHAN, M.K.** ; **ALLISON, J.P** ; **WOLCHOK, J.D.** Immune modulation in cancer with antibodies.. *Annu Rev Med*, 2014, vol. 65, 185-202
- **SCHIMMEL, P.** GTP hydrolysis in protein synthesis: two for Tu?. *Science*, 1993, vol. 259, 1264-1265
- **STEINMAN, R.M.** Decisions about dendritic cells: past, present, and future.. *Annu Rev Immunol*, 2012, vol. 30, 1-22
- **STEINMAN, R.M.** ; **HAWIGER, D.** ; **NUSSENZWEIG, M.C.** Tolerogenic dendritic cells.. *Annu Rev Immunol*, 2003, vol. 21, 685-711
- **SUGANYA, R.** ; **CHAKRABORTY, A.** ; **MIRIYALA, S.** ; **HAZRA, T.K.** ; **IZUMI, T.** Suppression of oxidative phosphorylation in mouse embryonic fibroblast cells deficient in apurinic/apyrimidinic endonuclease.. *DNA Repair (Amst)*, 2015, vol. 27, 40-48
- **VILLANI, A.C.** ; **SATIJA, R.** ; **REYNOLDS, G.** ; **SARKIZOVA, S.** ; **SHEKHAR, K.** ; **FLETCHER, J.** ; **GRIESBECK, M.** ; **BUTLER, A.** ; **ZHENG, S.** ; **LAZO, S. et al.** Single-cell RNA-seq reveals new types of human blood dendritic cells, monocytes, and progenitors.. *Science*, 2017, vol. 356
- **WALLACE, D.C.** ; **FAN, W.** ; **PROCACCIO, V.** Mitochondrial energetics and therapeutics.. *Annu Rev Pathol*, 2010, vol. 5, 297-348
- **WOLF, A.J.** ; **REYES, C.N.** ; **LIANG, W.** ; **BECKER, C.** ; **SHIMADA, K.** ; **WHEELER, M.L.** ; **CHO, H.C.** ; **POPESCU, N.I.** ; **COGGESHALL, K.M.** ; **ARDITI, M. et al.** Hexokinase Is an Innate Immune Receptor for the Detection of Bacterial Peptidoglycan.. *Cell*, 2016, vol. 166, 624-636
- **ZHANG, J.** ; **NUEBEL, E.** ; **WISIDAGAMA, D.R.** ; **SETOGUCHI, K.** ; **HONG, J.S.** ; **VAN HORN, C.M.** ; **IMAM, S.S.** ; **VERGNES, L.** ; **MALONE, C.S.** ; **KOEHLER, C.M. et al.** Measuring energy metabolism in cultured cells, including human pluripotent stem cells and differentiated cells.. *Nat Protoc*, 2012, vol. 7, 1068-1085
- **ZHANG, L.** ; **SU, J.** ; **XIE, Q.** ; **ZENG, L.** ; **WANG, Y.** ; **YI, D.** ; **YU, Y.** ; **LIU, S.** ; **LI, S.** ; **XU, Y.** 2-Deoxy-d-Glucose Sensitizes Human Ovarian Cancer Cells to Cisplatin by Increasing ER Stress and Decreasing ATP Stores in Acidic Vesicles.. *J Biochem Mol Toxicol*, 2015, vol. 29, 572-578

- **ZHANG, P.** ; **MCGRATH, B.** ; **LI, S.** ; **FRANK, A.** ; **ZAMBITO, F.** ; **REINERT, J.** ; **GANNON, M.** ; **MA, K.** ; **MCNAUGHTON, K.** ; **CAVENER, D.R**. The PERK eukaryotic initiation factor 2 alpha kinase is required for the development of the skeletal system, postnatal growth, and the function and viability of the pancreas.. *Mol Cell Biol*, 2002, vol. 22, 3864-3874